(19) Europäisches Patentamt
European Patent Office
Office européen des brevets

(11) EP 2 597 094 A1

(12) EUROPEAN PATENT APPLICATION
published in accordance with Art. 153(4) EPC

(43) Date of publication:
**29.05.2013 Bulletin 2013/22**

(21) Application number: **11803346.3**

(22) Date of filing: **08.07.2011**

(51) Int Cl.:
***C07D 471/04*** *(2006.01)* ***H01L 51/50*** *(2006.01)*

(86) International application number:
**PCT/JP2011/003920**

(87) International publication number:
**WO 2012/005009 (12.01.2012 Gazette 2012/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2010 JP 2010156623**

(71) Applicant: **Idemitsu Kosan Co., Ltd.**
**Chiyoda-ku**
**Tokyo 100-8321 (JP)**

(72) Inventors:
- **ITO, Hirokatsu**
  **Sodegaura-shi**
  **Chiba 299-0293 (JP)**
- **KAWAMURA, Masahiro**
  **Sodegaura-shi**
  **Chiba 299-0293 (JP)**
- **KAWAMURA, Yuichiro**
  **Sodegaura-shi**
  **Chiba 299-0293 (JP)**
- **MIZUKI, Yumiko**
  **Sodegaura-shi**
  **Chiba 299-0293 (JP)**
- **SAITO, Hiroyuki**
  **Sodegaura-shi**
  **Chiba 299-0293 (JP)**

(74) Representative: **Gille Hrabal**
**Patentanwälte**
**Brucknerstrasse 20**
**40593 Düsseldorf (DE)**

# (54) IMIDAZOPYRIDINE DERIVATIVES AND ORGANIC ELECTROLUMINESCENT ELEMENTS CONTAINING SAME

(57) An imidazopyridine derivative shown by the following formula (1).

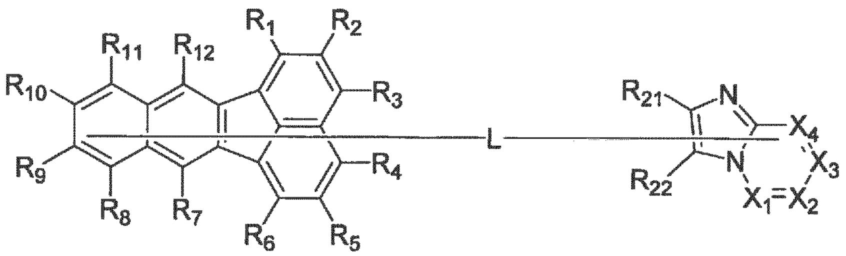

(1)

EP 2 597 094 A1

## Description

TECHNICAL FIELD

The invention relates to an imidazopyridine derivative, and an organic electroluminescence device including the same.

BACKGROUND ART

An organic electroluminescence (EL) device that utilizes an organic substance is a promising inexpensive solid-state emitting large full-color display, and has been extensively developed.
The organic EL device normally includes an emitting layer, and a pair of opposing electrodes holding the emitting layer therebetween. When an electric field is applied between the electrodes, electrons and holes are injected into the emitting layer respectively from the cathode and the anode. The electrons and the holes recombine in the emitting layer to produce an excited state, and the energy is emitted as light when the excited state returns to the ground state.
It is difficult to improve the performance of an organic EL device in which all of the hole-injecting function, the electron-injecting function, and the emitting function is implemented by a single layer. Therefore, the performance of an organic EL device is improved by providing a plurality of organic layers that differ in function between the electrodes. Specifically, a structure in which three or more layers such as a hole-transporting layer, an emitting layer, and an electron-transporting layer are stacked between two electrodes has been generally employed.
An organic EL device that was developed in an early stage was insufficient in terms of the drive voltage, the luminous efficiency, and the durability. Therefore, various technical improvements have been made to address this problem. For example, Patent Documents 1 to 3 implement a decrease in drive voltage by improving the electron-transporting material.
Patent Document 1 discloses a material that includes an anthracene skeleton. Patent Document 1 discloses various anthracene skeletons, but is silent about the properties of a benzofluoranthene skeleton, for example.
Patent Document 2 discloses various materials that include a benzimidazole skeleton. In Patent Document 3, the lifetime of the device is improved by utilizing a structure having a high glass transition temperature to improve the durability of a compound.
[Patent Document 1] JP-A-2004-2297
[Patent Document 2] KR-A-10-2009-0059849
[Patent Document 3] WO09/14826

DISCLOSURE OF THE INVENTION

An object of the invention is to provide an imidazopyridine derivative that makes it possible to decrease the drive voltage of an organic electroluminescence device, and provide the organic electroluminescence device with a high efficiency and a long lifetime.
The invention provides the following imidazopyridine derivative that makes it possible to decrease the drive voltage of an organic electroluminescence device, and provide the organic electroluminescence device with a high efficiency and a long lifetime.

1. An imidazopyridine derivative shown by a formula (1),

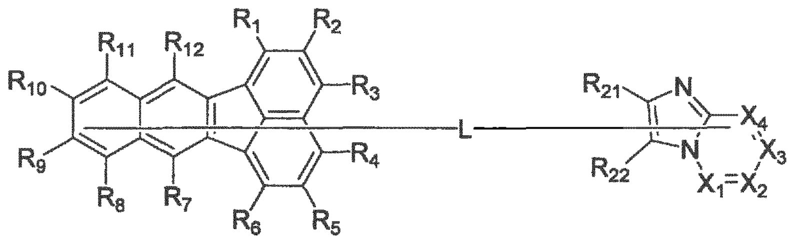

(1)

wherein one of $R_1$ to $R_{12}$ is a single bond that is bonded to L, the remainder of $R_1$ to $R_{12}$ are independently a hydrogen atom, a halogen atom, a cyano group, a substituted or unsubstituted alkyl group having 1 to 10 carbon atoms, a substituted or unsubstituted cycloalkyl group having 3 to 8 carbon atoms that form a ring (hereinafter

referred to as "ring carbon atoms"), a substituted or unsubstituted silyl group, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 atoms that form a ring (hereinafter referred to as "ring atoms"), $X_1$ to $X_4$ are independently a nitrogen atom or a linking group shown by $C(R_{23})$, and/or $X_1$ and $X_2$, $X_2$ and $X_3$, and $X_3$ and $X_4$ respectively bond to each other to form a substituted or unsubstituted aromatic ring having 6 to 18 ring carbon atoms or a substituted or unsubstituted aromatic heteroring having 5 to 18 ring atoms, provided that a plurality of $R_{23}$ may be either the same or different when a plurality of linking groups shown by $C(R_{23})$ are present, one of $R_{21}$ to $R_{23}$ is a single bond that is bonded to L, the remainder of $R_{21}$ to $R_{23}$ are independently a hydrogen atom, a halogen atom, a cyano group, a substituted or unsubstituted alkyl group having 1 to 10 carbon atoms, a substituted or unsubstituted cycloalkyl group having 3 to 8 ring carbon atoms, a substituted or unsubstituted silyl group, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 ring atoms, and L is a single bond, a substituted or unsubstituted arylene group having 6 to 30 ring carbon atoms, a substituted or unsubstituted divalent heterocyclic group having 5 to 30 ring atoms, or a linking group in which two or three of these divalent groups are bonded via a single bond:

2. The imidazopyridine derivative according to 1, the imidazopyridine derivative being shown by a formula (2),

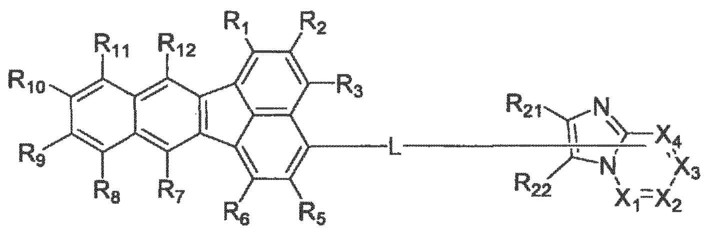
(2)

wherein $X_1$ to $X_4$, $R_1$ to $R_3$, $R_5$ to $R_{12}$, $R_{21}$ to $R_{23}$, and L are the same as defined for the formula (1).

3. The imidazopyridine derivative according to 1 or 2, the imidazopyridine derivative being shown by a formula (3),

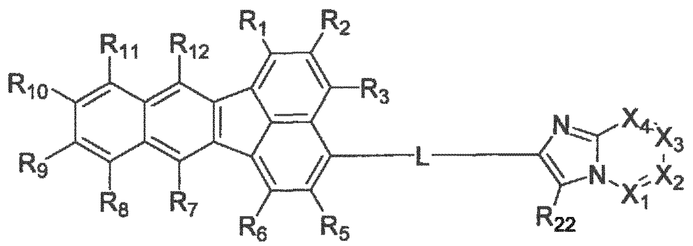
(3)

wherein $X_1$ to $X_4$, $R_1$ to $R_3$, $R_5$ to $R_{12}$, $R_{21}$, $R_{23}$, and L are the same as defined for the formula (1).

4. The imidazopyridine derivative according to any one of 1 to 3, wherein $R_7$ and $R_{12}$ are independently an aryl group having 6 to 30 ring carbon atoms.

5. The imidazopyridine derivative according to any one of 1 to 4, the imidazopyridine derivative being a material for an organic electroluminescence device.

6. The imidazopyridine derivative according to 5, wherein the material is an electron-injecting material or an electron-transporting material.

7. An organic electroluminescence device including a cathode, an anode, and one or more organic thin film layers that include at least an emitting layer and are provided between the cathode and the anode, at least one organic thin film layer among the one or more organic thin film layers including the imidazopyridine derivative according to any one of 1 to 4 either alone or as a component of a mixture.

8. The organic electroluminescence device according to 7, wherein the one or more organic thin film layers include an electron-injecting layer or an electron-transporting layer, and the electron-injecting layer or the electron-transporting layer includes the imidazopyridine derivative according to any one of 1 to 4 either alone or as a component of a mixture.

9. The organic electroluminescence device according to 8, wherein the electron-injecting layer or the electron-transporting layer that includes the imidazopyridine derivative further includes a reducing dopant.

10. The organic electroluminescence device according to 9, wherein the reducing dopant is one substance or two or more substances selected from the group consisting of alkali metals, alkaline-earth metals, rare earth metals,

alkali metal oxides, alkali metal halides, alkaline-earth metal oxides, alkaline-earth metal halides, rare earth metal oxides, rare earth metal halides, organic complexes of alkali metals, organic complexes of alkaline-earth metals, and organic complexes of rare earth metals.

11. The organic electroluminescence device according to 10, wherein the reducing dopant is an 8-quinolinol complex of an alkali metal.

The invention thus provides an imidazopyridine derivative that makes it possible to decrease the drive voltage of an organic electroluminescence device, and provide the organic electroluminescence device with a high efficiency and a long lifetime.

BRIEF DESCRIPTION OF THE DRAWINGS

FIG. 1 is a view showing an example of a first embodiment of the invention.

FIG 2A is a view showing the relationship between the energy gaps of respective layers.

FIG 2B is a view showing an effect based on the relationship between the energy gaps of respective layers.

MODE FOR CARRYING OUT THE INVENTION

An imidazopyridine derivative according to the invention is shown by the following formula (1):

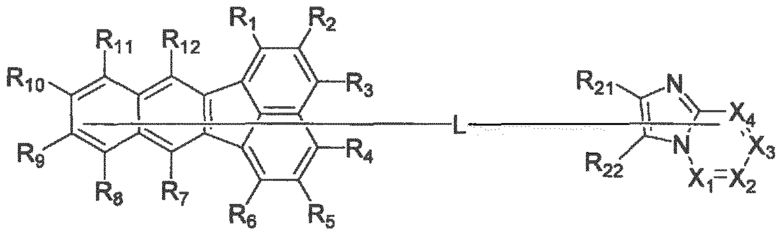

(1)

wherein one of $R_1$ to $R_{12}$ is a single bond that is bonded to L, the remainder of $R_1$ to $R_{12}$ are independently a hydrogen atom, a halogen atom, a cyano group, a substituted or unsubstituted alkyl group having 1 to 10 carbon atoms, a substituted or unsubstituted cycloalkyl group having 3 to 8 ring carbon atoms, a substituted or unsubstituted silyl group, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 ring atoms,

$X_1$ to $X_4$ are independently a nitrogen atom or a linking group shown by $C(R_{23})$, and/or $X_1$ and $X_2$, $X_2$ and $X_3$, and $X_3$ and $X_4$ respectively bond to each other to form a substituted or unsubstituted aromatic ring having 6 to 18 ring carbon atoms or a substituted or unsubstituted aromatic heteroring having 5 to 18 ring atoms,

a plurality of $R_{23}$ may be the same or different when a plurality of linking groups shown by $C(R_{23})$ are present,

one of $R_{21}$ to $R_{23}$ is a single bond that is bonded to L, and the remainder of $R_{21}$ to $R_{23}$ are independently a hydrogen atom, a halogen atom, a cyano group, a substituted or unsubstituted alkyl group having 1 to 10 carbon atoms, a substituted or unsubsfltuted cycloalkyl group having 3 to 8 ring carbon atoms, a substituted or unsubstituted silyl group, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 ring atoms, and

L is a single bond, a substituted or unsubstituted arylene group having 6 to 30 ring carbon atoms, a substituted or unsubstituted divalent heterocyclic group having 5 to 30 ring atoms, or a linking group in which two or three of these divalent groups are bonded via a single bond.

When a plurality of arylene groups or a plurality of heterocyclic groups are included in the linking group represented by L in the formula (1), the plurality of arylene groups or the plurality of heterocyclic groups may be the same or different

Since a benzofluoranthene skeleton has high planarity so that the molecules overlap advantageously, it is considered that the imidazopyridine derivative of the invention exhibits a high carrier-transporting capability. In particular, due to high planarity, a benzofluoranthene skeleton exhibits a carrier-transporting capability higher than that of a fluoranthene skeleton, for example.

Since a benzofluoranthene skeleton exhibits high carrier resistance, it is expected that an organic EL device that utilizes

the imidazopyridine derivative of the invention has an improved lifetime. For example, when using an electron-trapping dopant for the emitting layer of the organic EL device, holes may enter the electron-injecting layer. Since the imidazopyridine derivative of the invention that includes a benzofluoranthene skeleton exhibits hole resistance, it is considered that a deterioration in the device can be prevented.

The imidazopyridine derivative of the invention that includes a benzofluoranthene skeleton exhibits a high affinity (Af). When using the imidazopyridine derivative as a material for the electron-transporting layer, for example, the electron-transporting layer exhibits high interaction with a metal complex layer or a reducing dopant layer that is adjacent to the electron-transporting layer, so that an excellent electron-injecting capability is expected to be obtained.

The imidazopyridine derivative of the invention that includes a benzofluoranthene skeleton thus makes it possible to drive an organic EL device at a low voltage.

A nitrogen atom has a high electronegafivity, and a tendency to attract electrons of a carbon atom of a benzene ring or the like adjacent thereto. Imidazopyridine and benzimidazole are both a nitrogen-containing heterocyclic ring. Since imidazopyridine includes a nitrogen atom in the 6-membered ring, it is considered that imidazopyridine has a high electron-attracting effect on the carbon atoms of the 6-membered ring as compared with, for example, benzimidazole that does not include a nitrogen atom in the 6-membered ring. Therefore, electrical bias occurs within the molecule of imidazopyridine in which electrons are easily attracted by the nitrogen atom, so that affinity with a metal (cathode) increases due to an increase in polarity. It is considered that imidazopyridine thus exhibits a high electron-injecting capability.

A substituent is normally bonded to the nitrogen atom of benzimidazole, and may become steric hindrance when benzimidazole is coordinated to a metal. On the other hand, since imidazopyridine can reduce steric hindrance of the site coordinated to a metal, the electron-injecting capability can be improved as compared with benzimidazole.

It is considered that the imidazopyridine derivative of the invention that includes an imidazopyridine skeleton thus makes it possible to drive an organic EL device at a low voltage.

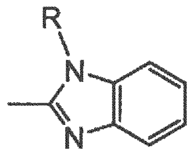

Benzimidazole

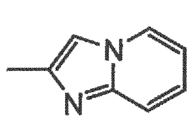

Imidazopyridine

The imidazopyridine derivative of the invention includes a benzofluoranthene skeleton and an imidazopyridine skeleton integrally, and exhibits an excellent carrier-transporting capability, excellent carrier resistance, and an excellent electron-injecting capability at the same time.

The imidazopyridine derivative represented by the formula (1) is preferably an imidazopyridine derivative represented by the following formula (2), and more preferably an imidazopyridine derivative represented by the fiollowing formula (3).

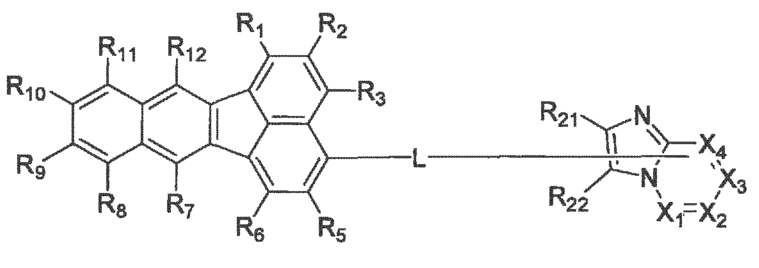

(2)

wherein $X_1$ to $X_4$, $R_1$ to $R_3$, $R_5$ to $R_{12}$, $R_{21}$ to $R_{23}$, and L are the same as defined for the formula (1).

In the imidazopyridine derivative shown by the formula (1), $R_3$ and $R_4$ are active sites. The stability of the imidazopyridine derivative tends to be improved by introducing a substituent into $R_3$ and $R_4$. The stability of the imidazopyridine derivative represented by the formula (2) is improved since $R_4$ is a single bond that is bonded to L.

When using for an organic EL device the imidazopyridine derivative represented by the formula (2) that exhibits improved stability, the organic EL device tends to have a further improved lifetime.

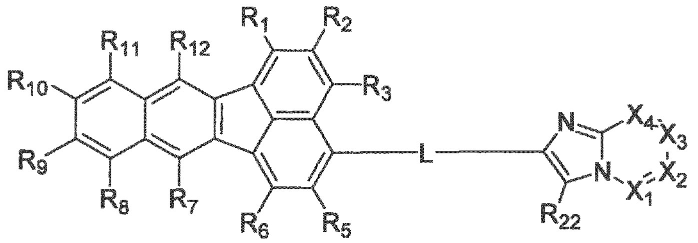

(3)

wherein $X_1$ to $X_4$, $R_1$ to $R_3$, $R_5$ to $R_{12}$, $R_{22}$, $R_{23}$, and L are the same as defined for the formula (1).

In the formula (3), $R_{21}$ is a single bond that is bonded to L. Therefore, when using the imidazopyridine derivative represented by the formula (3), for example, for an electron-transporting layer of an organic EL device, the electron-injecting capability from the electrode can be improved, so that it may be possible to still further decrease the drive voltage. Moreover, the carrier-transporting capability of the imidazopyridine derivative increases since steric hindrance of L and the imidazopyridine part decreases and planar molecular arrangement easily occurs, so that it may be possible to further decrease the drive voltage.

When L in the imidazopyridine derivatives of the invention represented by the formulas (1) to (3) (hereinafter may be referred to simply as "imidazopyridine derivative of the invention") is a single bond, it is considered that carrier transfer occurs smoothly within the molecule (i.e., electrons can promptly transfer to the carrier-transporting site) since the distance between the benzofluoranthene skeleton (carrier-transporting site) and the imidazopyridine skeleton (electron-injecting site) is short. Therefore, it is considered that the imidazopyridine derivative of the invention makes it possible to decrease the drive voltage when L is a single bond.

When L in the imidazopyridine derivative of the invention includes an arylene group and/or a divalent heterocyclic group, electronic involvement of the benzofluoranthene skeleton and the imidazopyridine skeleton can be reduced. Specifically, since the distance between the imidazopyridine skeleton and the benzofluoranthene skeleton increases, interference between the function of the electron-injecting site and the function of the carrier-transporting site can be reduced (although intramolecular carrier transfer may take time). Therefore, it may be possible to decrease the drive voltage due to the absence of interference during intermolecular carrier transfer.

In the imidazopyridine derivative of the invention, it is preferable that $R_7$ and $R_{12}$ be independently an aryl group having 6 to 30 (preferably 6 to 20, and more preferably 6 to 12) ring carbon atoms. It is particularly preferable that $R_7$ and $R_{12}$ be phenyl groups.

When $R_7$ and $R_{12}$ are independently an aryl group, the planarity of the benzofluoranthene skeleton is considered to be improved. As a result, the distance between the molecules can be reduced due to an increase in the degree of overlap between the molecules, so that the carrier-transporting capability of the imidazopyridine derivative can be improved.

Each substituent of the imidazopyridine derivative of the invention is described below.

Examples of the alkyl group having 1 to 10 (preferably 1 to 6, and more preferably 1 to 4) carbon atoms for $R_1$ to $R_{12}$ and $R_{21}$ to $R_{23}$ include an ethyl group, a methyl group, an i-propyl group, a n-propyl group, a s-butyl group, a t-butyl group, a pentyl group, a hexyl group, and the like. Among these, an ethyl group, a methyl group, an i-propyl group, and a t-butyl group are preferable.

Examples of the cycloalkyl group having 3 to 8 (preferably 3 to 6) ring carbon atoms for $R_1$ to $R_{12}$ and $R_{21}$ to $R_{23}$ include a cyclopropyl group, a cyclobutyl group, a cyclopentyl group, a cyclohexyl group, a 4-methylcyclohexyl group, and the like. Among these, a cyclobutyl group, a cyclopentyl group, and a cyclohexyl group are preferable.

The alkoxy group having 1 to 20 (preferably 1 to 10, and more preferably 1 to 6) carbon atoms for $R_1$ to $R_{12}$ and $R_{21}$ to $R_{23}$ is shown by -OY. Examples of Y include the above alkyl groups.

Examples of the aryl group having 6 to 30 (preferably 6 to 20, and more preferably 6 to 12) ring carbon atoms for $R_1$ to $R_{12}$ and $R_{21}$ to $R_{23}$ include a phenyl group, a naphthyl group, a phenanthryl group, a pyrenyl group, a biphenyl group, a terphenyl group, an anthryl group, a chrysenyl group, a benzophenanthryl group, a benzanthryl group, a benzochrysenyl group, a fluorenyl group, a fluoranthenyl group, a naphthacenyl group, and the like. Among these, a phenyl group, a naphthyl group, a phenanthryl group, a pyrenyl group, a biphenyl group, and a terphenyl group are preferable.

The aryloxy group having 6 to 20 (preferably 6 to 10) ring carbon atoms for $R_1$ to $R_{12}$ and $R_{21}$ to $R_{23}$ is shown by -OAr. Examples of Ar include the above aryl groups.

Examples of the heterocyclic group having 5 to 30 (preferably 5 to 20, and more preferably 5 to 12) ring atoms for $R_1$ to $R_{12}$ and $R_{21}$ to $R_{23}$ include a pyridinyl group, a pyrazinyl group, a pyrimidinyl group, a pyridazinyl group, a triadinyl group, an indolyl group, a quinolinyl group, an isoquinolinyl group, a quinoxalinyl group, an acridinyl group, a pyrrolidinyl group, a dioxanyl group, a piperidinyl group, a morpholyl group, a piperazinyl group, a carbazolyl group, a furanyl group, a thiophenyl group, an oxazolyl group, an isoxazolyl group, an oxadiazolyl group, a benzoxazolyl group, a thiazolyl group, a thiadiazolyl group, a benzothiophenyl group, a benzothiazolyl group, a triazolyl group, an imidazolyl group, a benzimidazolyl group, a benzofuranyl group, a dibenzofuranyl group, a pyrazolyl group, an indazolyl group, an

imidazopyridyl group, a tetrazolyl group, and the like. Among these, a pyridinyl group, a pyrazinyl group, a pyrimidinyl group, a pyridazinyl group, a triazinyl group, a benzofuranyl group, a dibenzofuranyl group, a furanyl group, a thiophenyl group, a benzothiophenyl group, and a benzothiazolyl group are preferable.

The silyl group for $R_1$ to $R_{12}$ and $R_{21}$ to $R_{23}$ may be an alkylsilyl group having 3 to 30 carbon atoms or an arylsilyl group having 8 to 30 carbon atoms.

Examples of the alkylsilyl group having 3 to 30 (preferably 3 to 20, and more preferably 3 to 10) carbon atoms include a trimethylsilyl group, a triethylsilyl group, a t-butyldimethylsilyl group, a vinyldimethytsilyl group, a propytdimethylsilyl group, and the like. Among these, a trimethylsilyl group and a t-butyldimethylsilyl group are preferable.

Examples of the arylsilyl group having 8 to 30 carbon atoms include a triphenylsilyl group, a phenyldimethylsilyl group, a t-butyldiphenylsilyl group, a tritolylsilyl group, a trixylylsilyl group, a trinaphthylsilyl group, and the like. Among these, a triphenylsilyl group and a phenyldimethylsilyl group are preferable.

Examples of the arylene group having 6 to 30 (preferably 6 to 20, and more preferably 6 to 12) ring carbon atoms for L include a phenylene group, a naphthylene group, a biphenylene group, a terphenylene group, an anthrylene group, a pentacenyrene group, a perylenyrene group, a pycenyrene group, a pyrenylene group, a pentaphenylene group, a fluorenylene group, a chrysenyrene group, a phenanthrylene group, and the like. Among these, a phenylene group, a naphthylene group, a biphenylene group, a terphenylene group, a fluorenylene group, and a phenanthrylene group are preferable.

Examples of the divalent heterocyclic group having 5 to 30 (preferably 5 to 20, and more preferably 5 to 12) ring atoms for L include a pyridinylene group, a pyradinylene group, a pyrimidinylene group, a pyridadinylene group, a triadinylene group, an indolinylene group, a quinolinylene group, an isoquinolinylene group, a quinoxalinylene group, an acridinylene group, a pyrrolidinylene group, a dioxanylene group, a piperidinylene group, a morpholilene group, a piperazinylene group, a carbazolylene group, a furanylene group, a thiophenylene group, an oxazolylene group, an isoxazolylene group, an oxadiazolylene group, a benzoxazolylene group, a thiazolylene group, a thiadiazolylene group, a benzothiophenylene group, a benzothiazolylene group, a triazolylene group, an imidazolylene group, a benzimidazolylene group, a puranylene group, a dibenzofuranylene group, a dibenzothiophenylene group, a pyrazolylene group, an indazolylene group, an imidazopyridylene group, a tetrazolylene group, and the like. Among these, a pyridinylene group, a pyradinylene group, a pyrimidinylene group, a pyridadinylene group, a triadinylene group, a dibenzofuranylene group, and a dibenzothiophenylene group are preferable.

Examples of the halogen atom include a fluorine atom, a chlorine atom, a bromine atom, an iodine atom, and the like. Among these, a fluorine atom is preferable.

When each substituent represented by $R_1$ to $R_{12}$, $R_{21}$ to $R_{23}$, and L additionally has a substituent, examples of the substituent include the above alkyl group, an alkylsilyl group, a halogenated alkyl group, an aryl group, a cycloalkyl group, an alkoxy group, a heterocyclic group, an aralkyl group, an aryloxy group, an arylthio group, an alkoxycarbonyl group, a halogen atom, a hydroxyl group, a nitro group, a cyano group, a carboxyl group, a carbonyl group, a dibenzofuranyl group, a fluorenyl group, and the like.

Note that the term "unsubstituted" used in connection with each substituent of the imidazopyridine derivative of the invention refers to substitution with a hydrogen atom. The term "hydrogen atom" used herein includes light hydrogen and deuterium.

The term "ring carbon atom" used herein refers to a carbon atom that forms a saturated ring, an unsaturated ring, or an aromatic ring. The term "ring atom" used herein refers to a carbon atom or a heteroatom that forms a ring (including a saturated ring, an unsaturated ring, and an aromatic ring).

Specific examples of the imidazopyridine derivative of the invention are shown below.

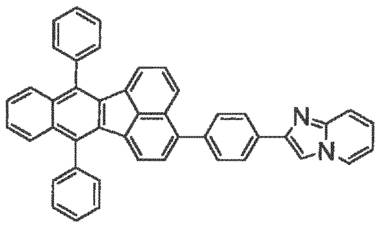

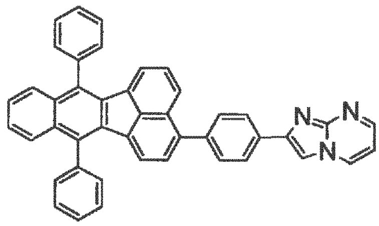

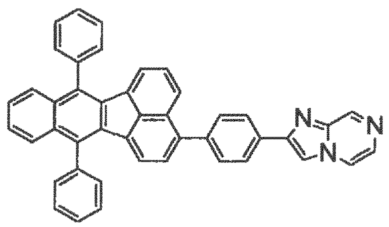

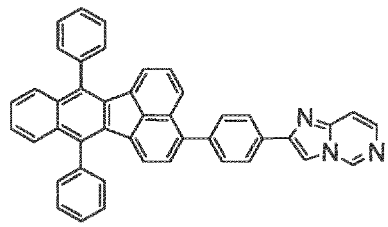

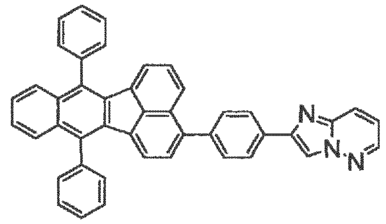

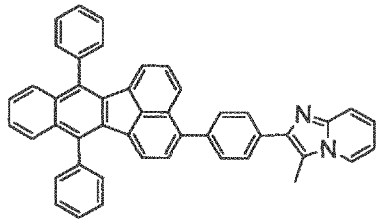

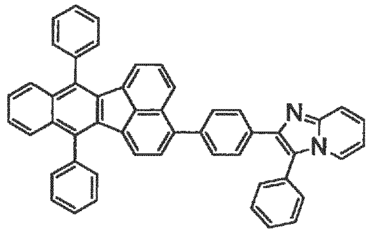

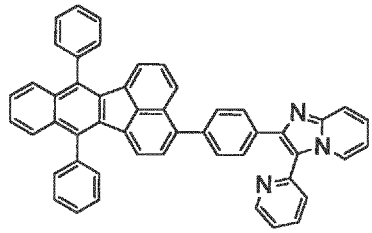

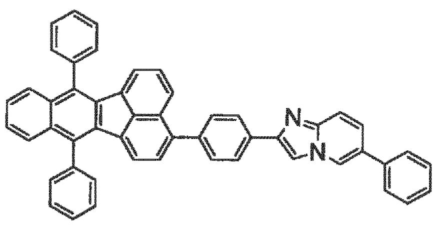

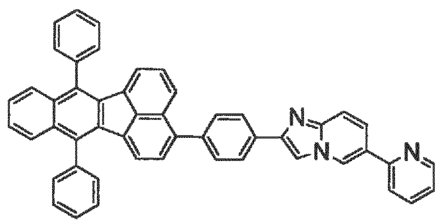

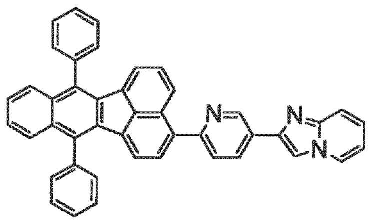

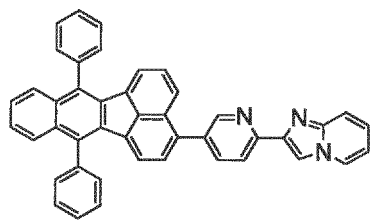

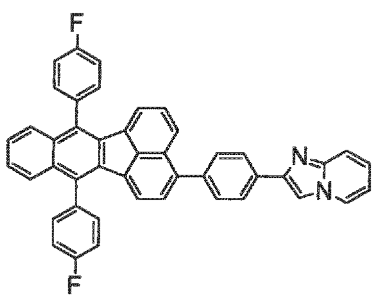

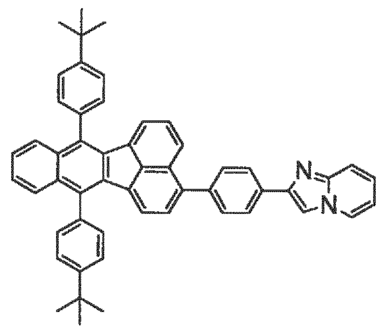

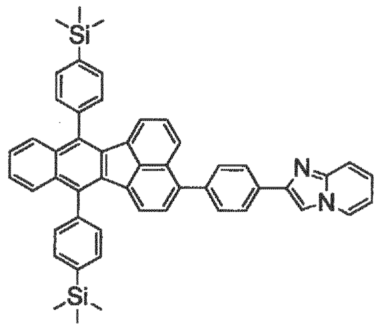

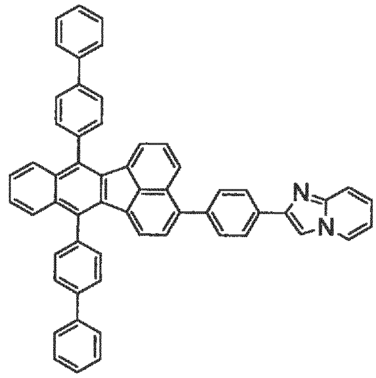

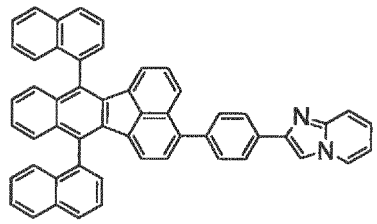

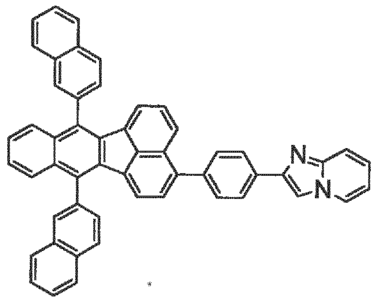

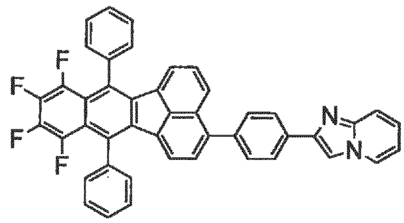

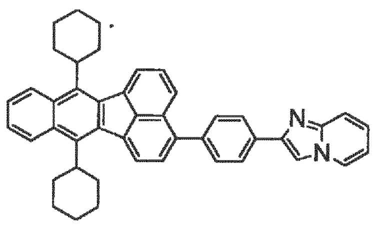

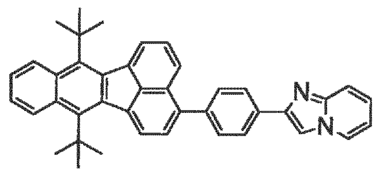

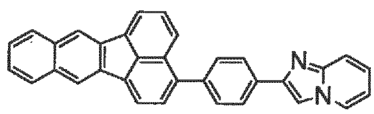

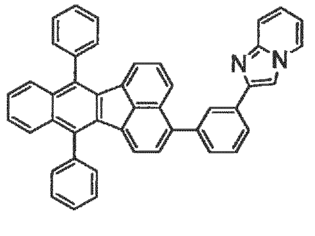

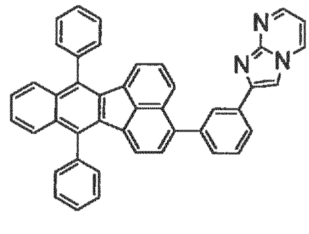

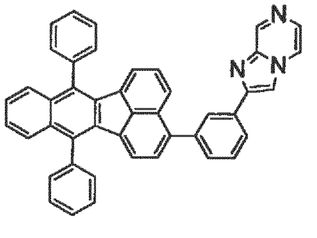

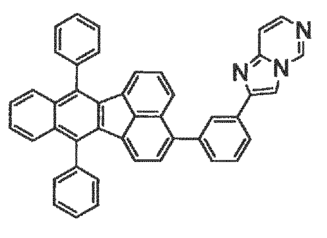

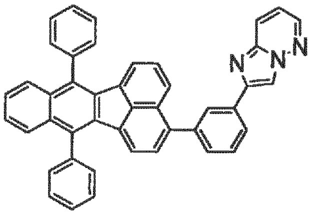

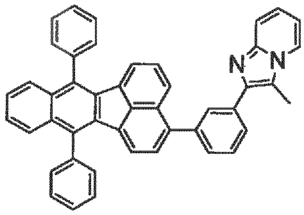

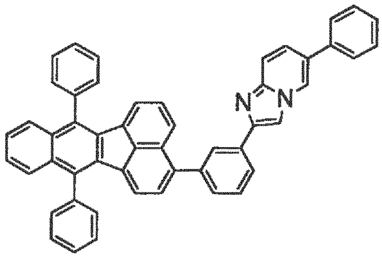

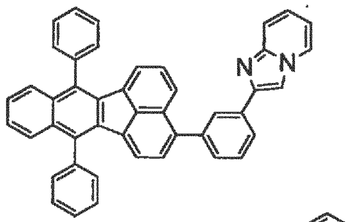

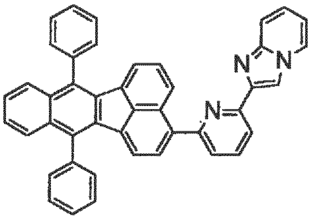

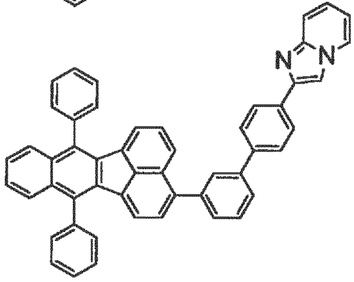

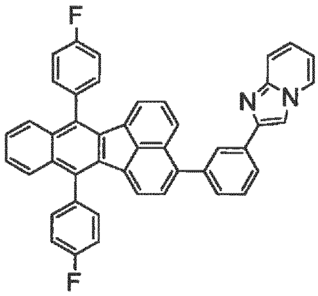

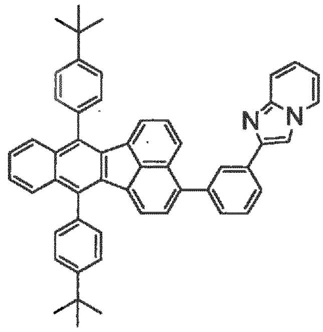

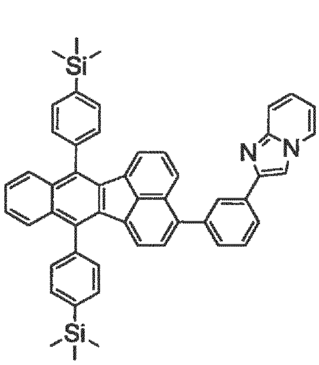

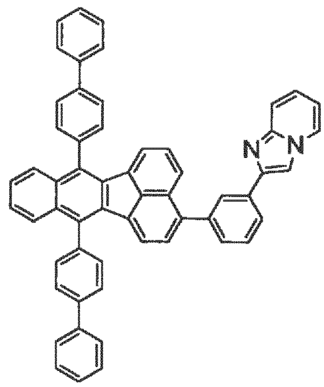

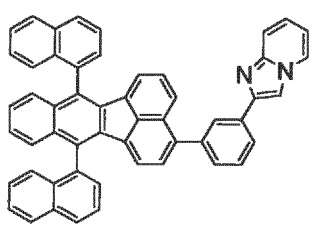

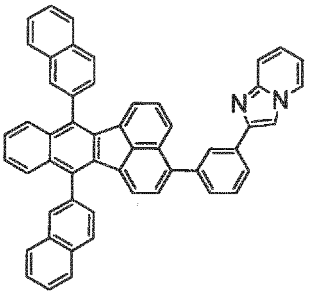

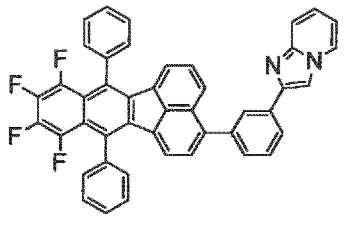

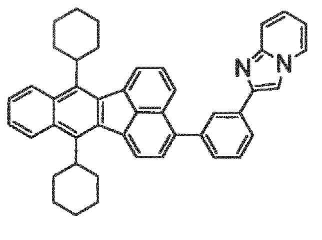

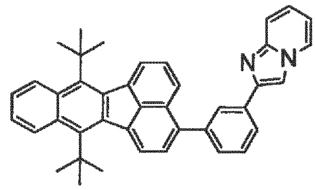

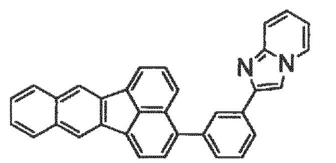

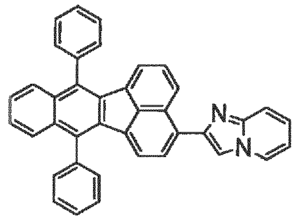

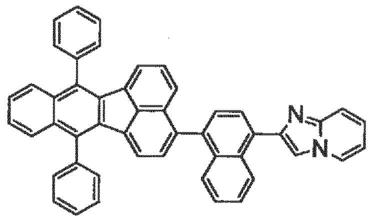

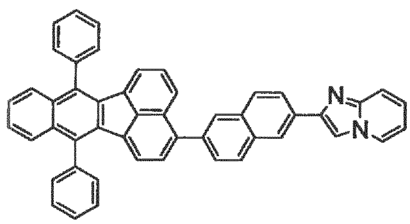

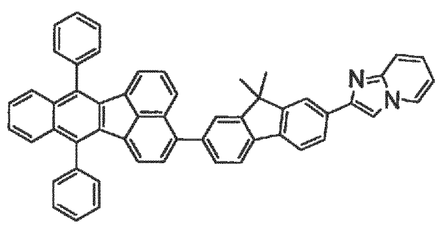

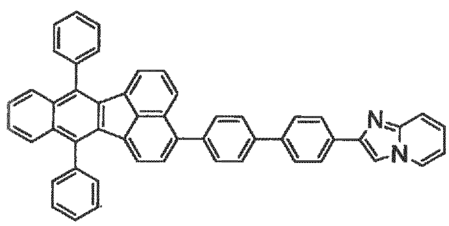

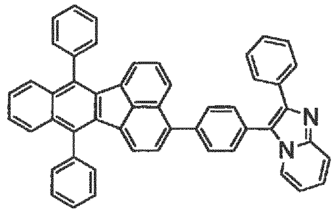

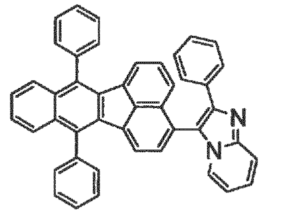

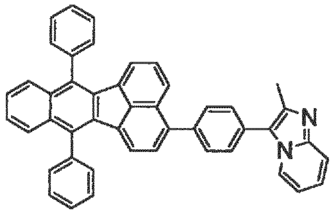

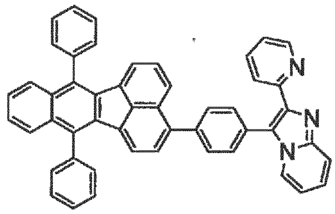

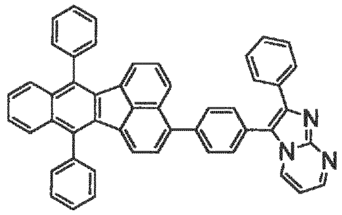

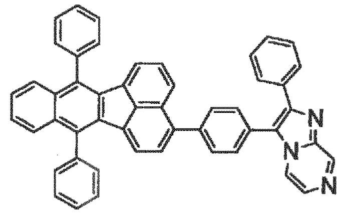

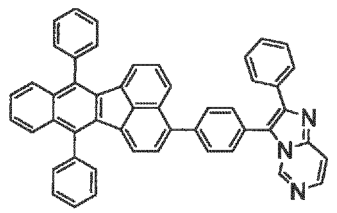

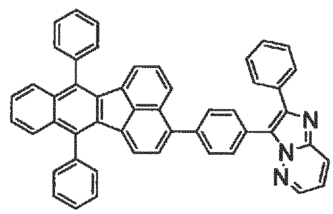

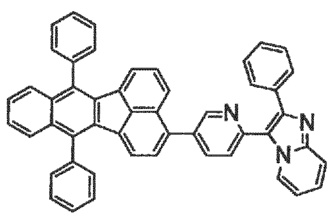

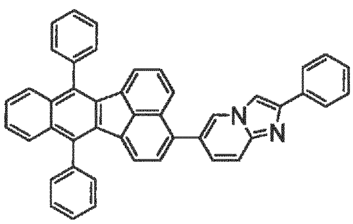

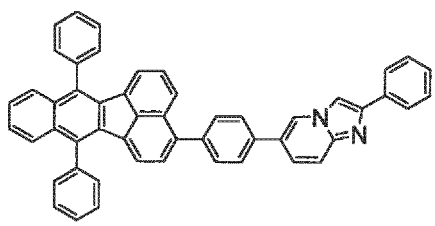

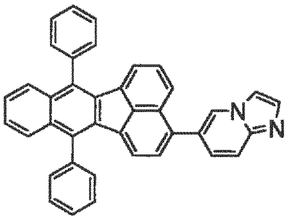

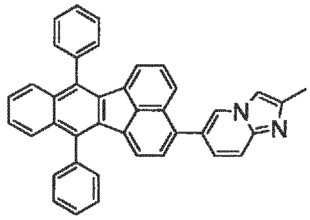

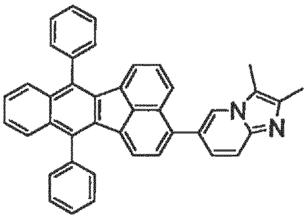

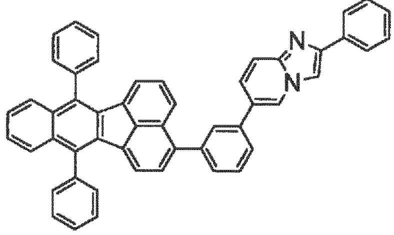

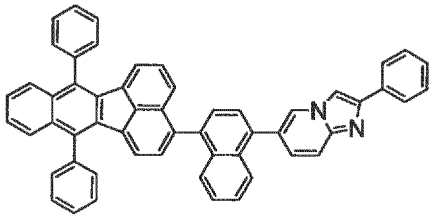

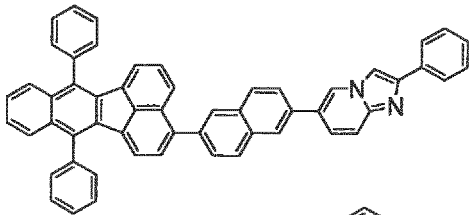

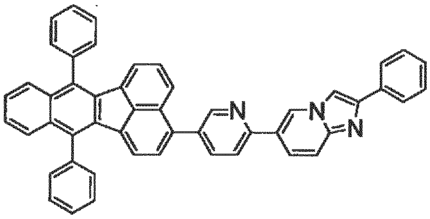

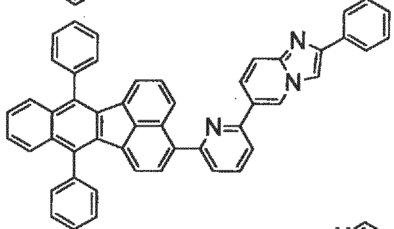

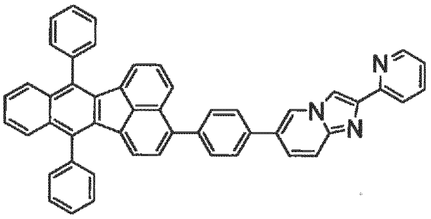

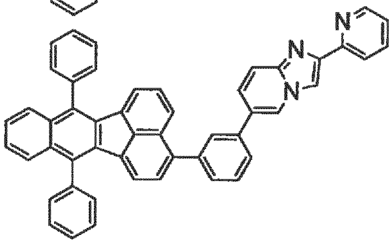

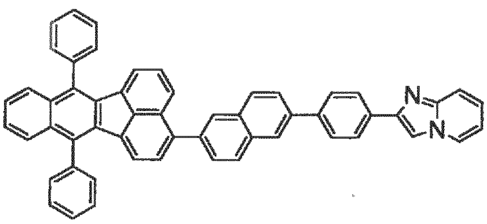

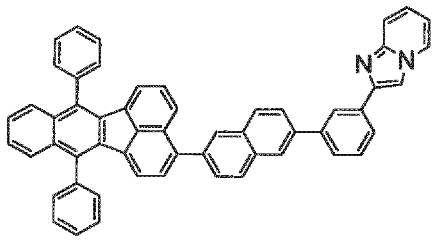

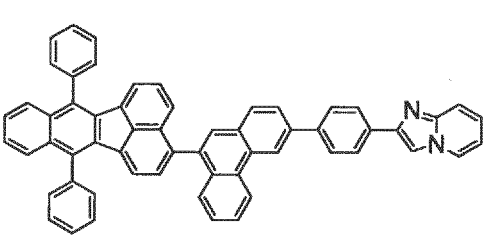

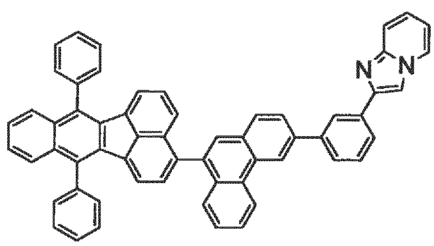

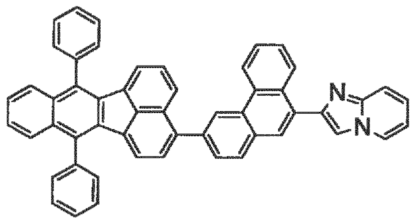
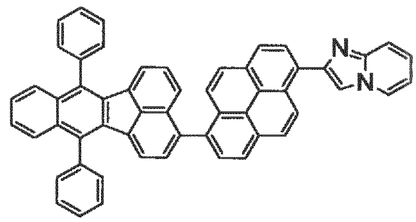

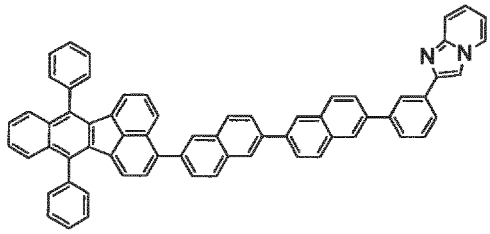
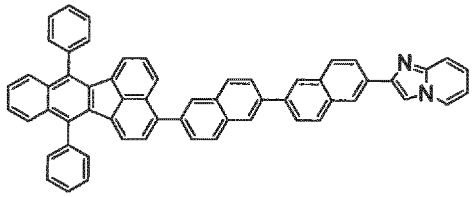

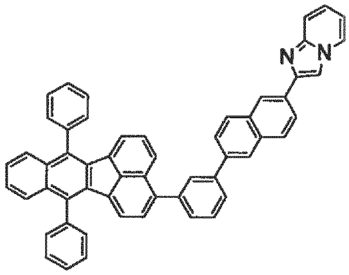
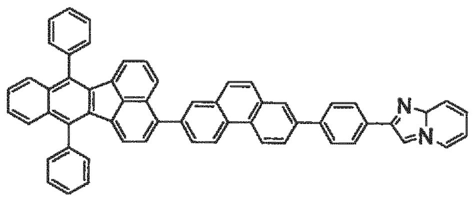

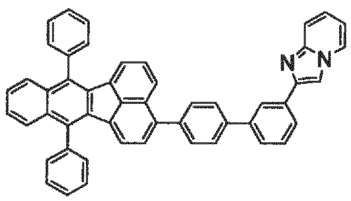
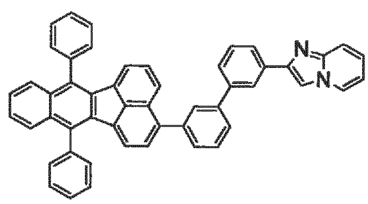

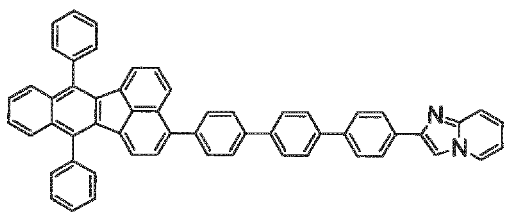
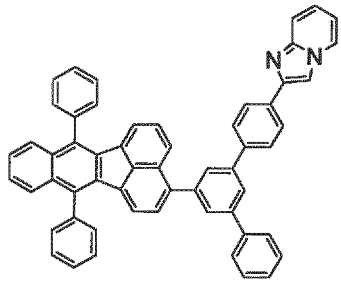

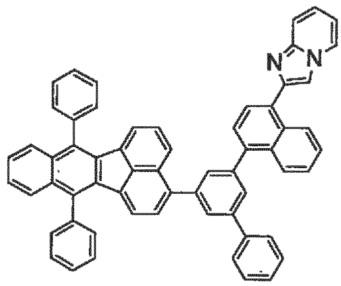
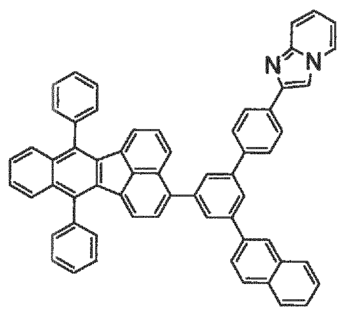

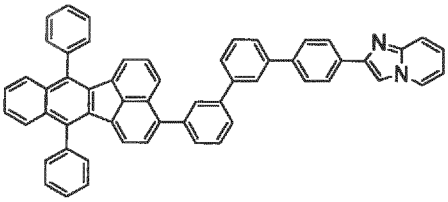
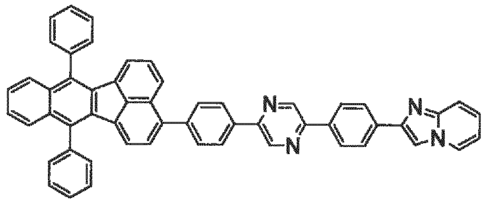

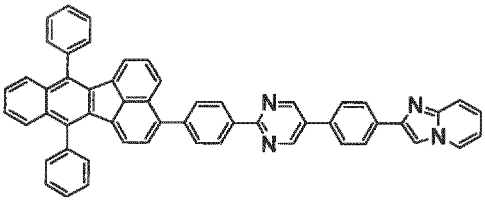
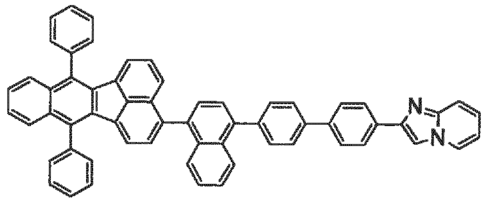

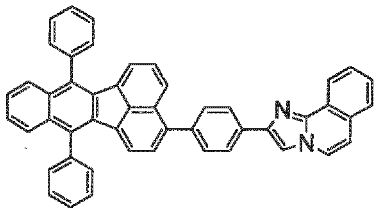
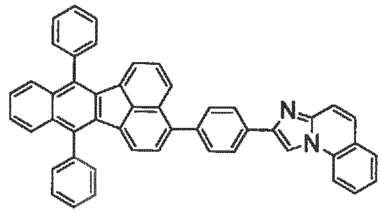

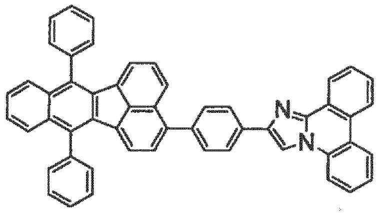
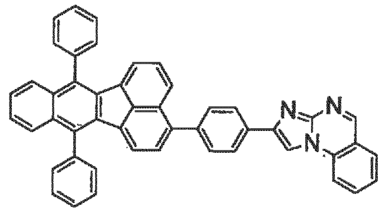

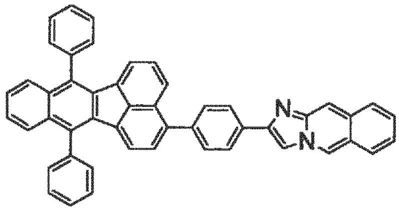
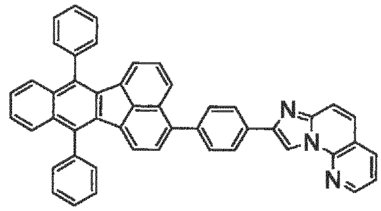

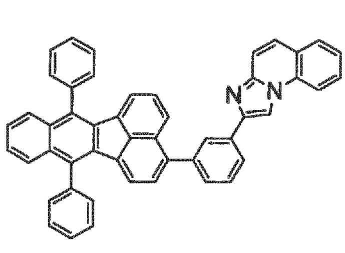
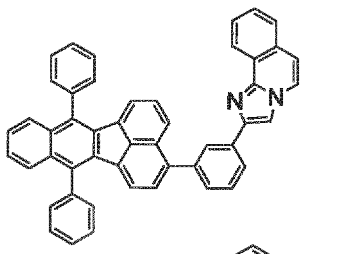

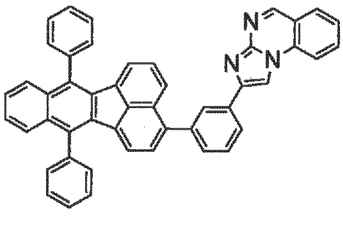
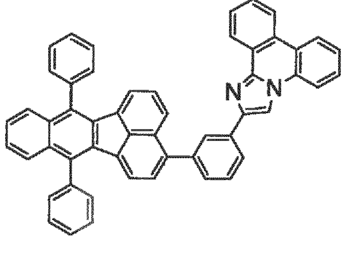

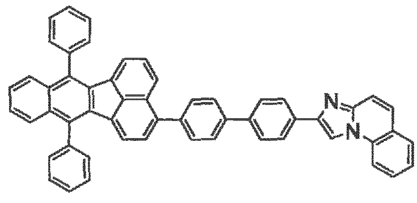
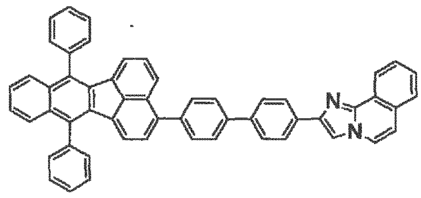

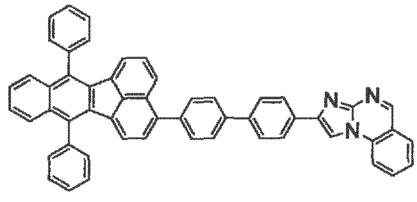
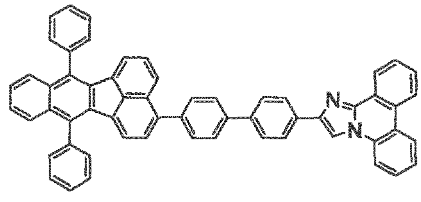

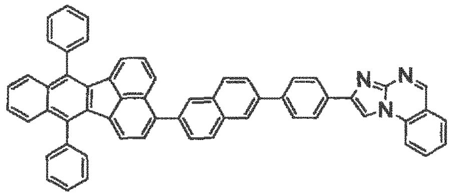
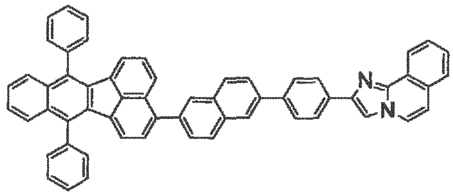

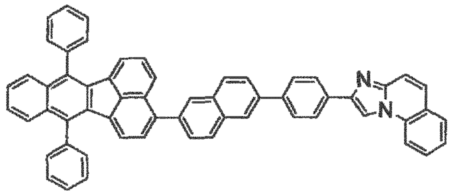
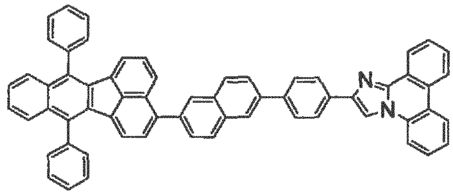

The imidazopyridine derivative of the invention is preferably used as a material for an organic EL device, and more preferably used as an electron-injecting material or an electron-transporting material for an organic EL device. This is because the imidazopyridine group mediates transfer of electrons from the adjacent layer. The imidazopyridine derivative of the invention may also be suitably used as a triplet energy barrier material for the reasons described below.

The benzofluoranthene skeleton that is the basic skeleton of the imidazopyridine derivative of the invention has high triplet energy, and exhibits a high triplet exciton confinement effect. Therefore, it is considered that a triplet-triplet fusion (TTF) phenomenon can be promoted, for example, by utilizing the imidazopyridine derivative as a material for a barrier layer of an organic EL device that is in contact with an emitting layer. The benzofluoranthene skeleton that is the basic skeleton of the imidazopyridine derivative of the invention improves molecular stacking within a thin film due to high planarity, so that the electron-transporting capability is improved. This may make it possible to promote injection of electrons into the emitting layer and improve the recombination efficiency in the emitting layer, so that the TTF phenomenon may efficiently occur. Since the imidazopyridine derivative of the invention includes an imidazopyridine skeleton that is a nitrogen-containing heterocyclic ring that exhibits a high electron-injecting capability from a metal-containing layer (e.g., electrode), an organic EL device having a lower drive voltage may be implemented without further stacking an electron-injecting layer.

The imidazopyridine derivative of the invention is thus a compound which exhibits both an electron-injecting/transporting function and a triplet energy barrier function (triplet barrier function).

The imidazopyridine derivative of the invention includes a structural site that exhibits a triplet barrier function (triplet barrier structural site) and a structural site that exhibits an electron-injecting/transporting function, as described above. The term "structural site" used herein refers to a cyclic structure (monocyclic structure or fused polycyclic structure excluding a substituent) included in the compound.

The term "triplet barrier structural site" used herein refers to a structural site included in the compound that has the lowest (smallest) triplet energy. Specifically, the triplet barrier structural site mainly determines the triplet energy of the compound. The triplet energy of the triplet barrier structural site refers to the triplet energy of an independent cyclic structure that is not substituted with a substituent and includes a hydrogen atom at a bonding position between structural sites. The triplet barrier structural site must be a fused polycyclic aromatic hydrocarbon compound. The reason therefor

is described below.

The transition state of a fused hydrocarbon ring is based on the π-π* transition in which the π electron cloud of the cyclic structure is involved. The π electron cloud has a narrow distribution, and has a small effect on the excited state of the emitting layer. When the structural site includes an unshared electron pair, a strong interaction with triplet excitons formed in the emitting layer occurs due to the unshared electron pair, and deactivation of triplet excitons formed by the host is promoted. This makes it difficult to cause the TTF phenomenon to efficiently occur. Therefore, the triplet barrier structural site of the barrier material is preferably a fused hydrocarbon ring that mainly forms an excited triplet state based on the π-π* transition.

When using the imidazopyridine derivative of the invention as the barrier material, it is preferable that the triplet energy of the barrier material be larger than the triplet energy of the host included in the emitting layer.

The triplet barrier function of the barrier material is mainly determined by the triplet barrier structural site. In general, when the energy of triplet excitons formed in the emitting layer is transferred to the adjacent barrier material, it is transferred to the structural site included in the barrier material that has the lowest triplet energy. When the triplet barrier structural site that has the lowest triplet energy is a fused polycyclic aromatic hydrocarbon compound, the barrier material effectively exhibits the triplet barrier function. Therefore, when the structural site included in the compound that has the lowest triplet energy is not formed of a carbon atom and a hydrogen atom, the compound does not include the triplet barrier structural site.

The TTF phenomenon is briefly described below.

When a voltage is applied to an organic EL device, holes and electrons are injected into the emitting layer respectively from the anode and the cathode, and recombine in the emitting layer to form excitons. In this case, singlet excitons and triplet excitons are formed in a ratio of 25:75 (%). In conventional fluorescent devices, light is emitted when singlet excitons return to the ground state, and triplet excitons return to the ground state via a thermal deactivation process without emitting light. According to S.M. Bachilo et al. (J. Phys. Chem. A, 104, 7711 (2000)), 1/5th of triplet excitons that account for 75% of the excitons initially formed change into singlet excitons.

The TTF phenomenon is a phenomenon in which singlet excitons are formed due to collision and fusion between triplet excitons. By utilizing the TTF phenomenon, singlet excitons formed due to collision and fusion between triplet excitons can be utilized to emit light in addition to singlet excitons initially formed in a ratio of 25%, so that the luminous efficiency of the device can be improved.

It is necessary to confine triplet excitons having a remarkably long lifetime as compared with singlet excitons within the emitting layer in order to cause the TTF phenomenon to efficiently occur.

It is preferable that the barrier layer that includes the imidazopyridine derivative of the invention be provided in contact with the emitting layer of a fluorescent device. It is considered that the TTF phenomenon occurs as a result of utilizing the barrier layer that includes the imidazopyridine derivative of the invention for a fluorescent device, so that a highly efficient organic EL device can be implemented.

Note that the term "barrier layer" used herein refers to a layer that exhibits a triplet energy barrier function, and differs in function from a hole barrier layer and a carrier barrier layer.

It is preferable that the barrier layer, the electron-injecting layer, or the electron-transporting layer that includes the imidazopyridine derivative of the invention further include a reducing dopant.

Examples of the reducing dopant include donor metals, donor metal compounds, and donor metal complexes. These reducing dopants may be used singly or in combination.

Note that the term "reducing dopant" used herein refers to a material that donates electrons (also referred to as "electron donor material"). The electron donor material is a material that interacts with an organic material that is included in the barrier layer, the electron-injecting layer, or the electron-transporting layer together with the electron donor material, or with an organic material that is included in a layer in contact with the barrier layer, the electron-injecting layer, or the electron-transporting layer, and that produces radical anions, or a material that includes an electron donor radical.

The term "donor metal" used herein refers to a metal that has a work function of 3.8 eV or less. The donor metal is preferably an alkali metal, an alkaline-earth metal, or a rare earth metal, and more preferably Cs, Li, Na, Sr, K, Mg, Ca, Ba, Yb, Eu, or Ce.

The term "donor metal compound" used herein refers to a compound that includes the donor metal above. The donor metal compound is preferably a compound that includes an alkali metal, an alkaline-earth metal, or a rare earth metal, and more preferably a halide, an oxide, a carbonate, or a borate of these metals. For example, the donor metal compound is a compound shown by MOx (wherein M is a donor metal, and x is 0.5 to 1.5), MFx (x is 1 to 3), or $M(CO_3)x$ (x is 0.5 to 1.5).

The term "donor metal complex" refers to a complex of the donor metal above. The donor metal complex is preferably an organic metal complex of an alkali metal, an alkaline-earth metal, or a rare earth metal. The donor metal complex is preferably an organic metal complex represented by the following formula (I):

$$M\left(Q\right)_n \qquad (I)$$

wherein M is a donor metal, Q is a ligand (preferably a carboxylic acid derivative, a diketone derivative, or a quinolinic derivative), and n is an integer of 1 to 4.

Specific examples of the donor metal complex include the tungsten paddlewheel disclosed in JP-A-2005-72012, and the like. The phthalocyanine compound disclosed in JP-A-11-345687 in which the central metal is an alkali metal or an alkaline-earth metal, may also be used as the donor metal complex.

The reducing donor is preferably one substance or two or more substances selected from the group consisting of alkali metals, alkaline-earth metals, rare earth metals, alkali metal oxides, alkali metal halides, alkaline-earth metal oxides, alkaline-earth metal halides, rare earth metal oxides, rare earth metal halides, organic complexes of alkali metals, organic complexes of alkaline-earth metals, and organic complexes of rare earth metals, and more preferably an 8-quinolinol complex of an alkali metal.

When utilizing the TTF phenomenon, the triplet energy of the compound which forms the barrier layer composed of imidazopyridine derivative of the invention must be higher than the triplet energy of the host that mainly forms the emitting layer. It is preferable that the imidazopyridine derivative of the invention included in the barrier layer and the host and the dopant included in the emitting layer satisfy the following formulas (1) and (2):

$$E^Tb > E^Th \qquad (1)$$

$$E^Td > E^Th \qquad (2)$$

where, $E^Th$ is the triplet energy of the host material, $E^Tb$ is the triplet energy of the nitrogen-containing heterocyclic derivative included in the barrier layer, and $E^Td$ is the triplet energy of the dopant.

FIG. 1 is a schematic configuration diagram showing an example of an organic EL device of a first embodiment of the invention. FIG. 2A schematically shows the lowest excited singlet energy and the lowest excited triplet energy of each layer. Note that the term "triplet energy" used herein refers to the difference between the energy in the lowest excited triplet state and the energy in the ground state, and the term "singlet energy" (may be referred to as "energy gap") used herein refers to the difference between the energy in the lowest excited singlet state and the energy in the ground state.

The organic EL device of the invention includes an anode, an emitting layer, an electron-transporting region, and a cathode sequentially. The organic EL device shown in FIG. 1 has a configuration in which an anode 10, a hole-transporting region 50, an emitting layer 20, an electron-transporting region 30, and a cathode 40 are stacked in this order. It is preferable to provide the hole-transporting region 50 between the anode 10 and the emitting layer 20. FIG. 2A shows an example in which the electron-transporting region includes only a barrier layer. Note that the electron-transporting region may also include an electron-injecting layer that has a higher electron injection capability. The electron-injecting layer may be formed using a compound (preferably a heteroring compound) that has been generally used to form the electron-injecting layer.

As shown in FIG. 2A, holes are injected into the emitting layer from the anode via the hole-transporting region, and electrons are injected into the emitting layer from the cathode via the electron-transporting region. The holes and the electrons recombine in the emitting layer to form singlet excitons and triplet excitons. The holes and the electrons may recombine on the molecules of the host, or may recombine on the molecules of the dopant. As shown in FIG. 2A, when the triplet energy of the host is referred to as $E^T_h$, and the triplet energy of the dopant is referred to as $E^T_d$, the relationship "$E^T_h < E^T_d$" is preferably satisfied. When the above relationship is satisfied, triplet excitons formed due to recombination of the holes and the electrons on the host do not transfer to the dopant that has a larger triplet energy (see FIG. 2B).

Triplet excitons formed due to recombination of the holes and the electrons on the molecules of the dopant promptly transfer to the molecules of the host. Specifically, triplet excitons formed on the host efficiently collide on the host due to the TTF phenomenon without transferring to the dopant, thus forming singlet excitons. Since the singlet energy $E^sd$ of the dopant is smaller than the singlet energy $E^sh$ of the host, the singlet excitons formed due to the TTF phenomenon transfer from the host to the dopant, and contribute to fluorescent emission of the dopant. In a dopant used for a fluorescent device, a transition from the excited triplet state to the ground state is forbidden, and triplet excitons are not optically deactivated, but are thermally deactivated. However, when the triplet energy of the host and the triplet energy of the dopant satisfy the above relationship, triplet excitons efficiently collide to form singlet excitons before

thermal deactivation, so that the luminous efficiency is improved.

It is preferable that the electron-transporting region include the barrier layer in an area adjacent to the emitting layer. The barrier layer prevents diffusion of triplet excitons formed in the emitting layer into the electron-transporting region, and increases the density of the triplet excitons in the emitting layer by confining the triplet excitons within the emitting layer. As a result, the TTF phenomenon efficiently occurs.

As shown in FIGS. 2A and 2B, it is preferable that the triplet energy $E^Tb$ of the compound that forms the barrier layer be larger than the triplet energy $E^Th$ and the triplet energy $E^Td$ in order to prevent diffusion of triplet excitons. Since the barrier layer prevents diffusion of triplet excitons formed in the emitting layer into the electron-transporting region, triplet excitons of the host efficiently form singlet excitons within the emitting layer. The singlet excitons thus formed transfer to the dopant, and are optically deactivated. The imidazopyridine derivative of the invention is used as a material that forms the barrier layer. Since the imidazopyridine derivative of the invention deteriorates to only a small extent due to hole resistance, the lifetime of the device increases.

Since the barrier layer that includes the imidazopyridine derivative of the invention also has an electron-injecting/transporting function, electrons injected into the barrier material move to a structural site that more easily donates electrons via the electron-transporting structural site. Specifically, the electrons move to a structural site that has a high LUMO level, and contribute to injection of electrons into the emitting layer.

A small-work-function metal-containing layer may be provided between the electron-transporting region and the cathode. The term "small-work-function metal-containing layer" used herein refers to a layer that includes a small-work-function metal or a small-work-function metal compound. The small-work-function metal-containing layer may be formed only of a small-work-function metal or a small-work-function metal compound, or may be formed by adding a small-work-function metal, a small-work-function metal compound, or a small-work-function metal complex as a donor to a material used to form the electron-transporting layer. The term "small-work-function metal" used herein refers to a metal that has a work function of 3.8 eV or less. Examples of the metal that has a work function of 3.8 eV or less include alkali metals, alkaline-earth metals, and the like. Examples of the alkali metals include Li, Na, K, Cs, and the like. Examples of the alkaline-earth metals include Mg, Ca, Sr, Ba, and the like. Further examples of the metal that has a work function of 3.8 eV or less include Yb, Eu, Ce, and the like. Examples of a preferable small-work-function metal compound include oxides, halides, carbonates, and borates of the small-work-function metals. Examples of the halides of the small-work-function metals include fluorides, chlorides, and bromides of the small-work-function metals. Among these, fluorides of the small-work-function metals are preferable. For example, LiF is preferably used. Examples of a preferable small-work-function metal complex include organic complexes of alkali metals, alkaline-earth metals, and rare earth metals.

The efficiency of a blue fluorescent layer is remarkably improved by utilizing the TTF phenomenon. Note that the luminous efficiency of a green fluorescent layer or a red fluorescent layer can also be improved by confining the triplet energy within the emitting layer.

In the organic EL device of the invention, it is preferable that the emitting layer include at least one of an anthracene derivative shown by the following formula (4) and a pyrene derivative shown by the following formula (5) as the host.

(Anthracene derivative)

The anthracene derivative is shown by the following formula (4):

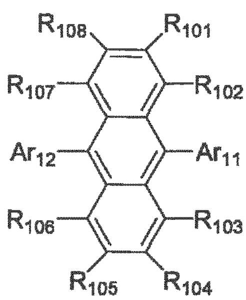

(4)

wherein $Ar_{11}$ and $Ar_{12}$ are independently a substituted or unsubstituted monocyclic group having 5 to 50 ring atoms, a substituted or unsubstituted fused cyclic group having 8 to 50 ring atoms, or a combination of the monocyclic group and the fused cyclic group, and

$R_{101}$ to $R_{108}$ are independently a group selected from a hydrogen atom, a substituted or unsubstituted monocyclic group having 5 to 50 ring atoms, a substituted or unsubstituted fused cyclic group having 8 to 50 ring atoms, a combination of

the monocyclic group and the fused cyclic group, a substituted or unsubstituted alkyl group having 1 to 50 carbon atoms, a substituted or unsubstituted cycloalkyl group having 3 to 50 ring carbon atoms, a substituted or unsubstituted alkoxy group having 1 to 50 carbon atoms, a substituted or unsubstituted aralkyl group having 7 to 50 carbon atoms, a substituted or unsubstituted aryloxy group having 6 to 50 ring carbon atoms, a substituted or unsubstituted silyl group, a halogen atom, and a cyano group.

The term monocyclic group" in the formula (4) refers to a group that includes only a cyclic structure that does not have a fused structure.

Specific examples of a preferable monocyclic group having 5 to 50 (preferably 5 to 30, and more preferably 5 to 20) ring atoms include aromatic groups such as a phenyl group, a biphenyl group, a terphenyl group, a quarter phenyl group, and the like, and heterocyclic groups such as a pyridyl group, pyrazyl group, pyrimidyl group, triazinyl group, furyl group, and thienyl group).

Among these, a phenyl group, a biphenyl group, and a terphenyl group are preferable.

The term "fused cyclic group" in the formula (4) refers to a group in which two or more cyclic structures (rings) are fused.

Specific examples of a preferable fused cyclic group having 8 to 50 (preferably 8 to 30, and more preferably 8 to 20) ring atoms include fused aromatic ring groups such as a naphthyl group, a phenanthryl group, an anthryl group, a chrysenyl group, a benzanthryl group, a benzophenanthryl group, a triphenylenyl group, a benzochrysenyl group, an indenyl group, a fluorenyl group, a 9,9-dimethylfluorenyl group, a benzofluorenyl group, a dibenzofluorenyl group, a fluoranthenyl group, a benzofluoranthenyl group, and the like, and fused heterocyclic groups such as a benzofuranyl group, a benzothiophenyl group, an indolyl group, a dibenzofuranyl group, a dibenzothiophenyl group, a carbazolyl group, a quinolyl group, and a phenanthrolinyl group).

Among these, a naphthyl group, a phenanthryl group, an anthryl group, a 9,9-dimethylfluorenyl group, a fluoranthenyl group, a benzanthryl group, a dibenzothiophenyl group, a dibenzofuranyl group, and a carbazolyl group are preferable.

Specific examples of the alkyl group having 1 to 50 carbon atoms, the cycloalkyl group having 3 to 50 ring carbon atoms, the alkoxy group having 1 to 50 carbon atoms, the aryloxy group having 6 to 50 ring carbon atoms, and the substituted or unsubstituted silyl group include those mentioned above in connection with the formula (1).

As an aralkyl group having 7 to 50 carbon atoms, an aralkyl group is shown by -Y-Z. Examples of Y include alkylene groups that correspond to the above alkyl groups. Examples of Z include the above aryl groups. The number of carbon atoms of the aralkyl group is preferably 7 to 50 (the number of carbon atoms of the aryl moiety is 6 to 49 (preferably 6 to 30, more preferably 6 to 20, and particularly preferably 6 to 12), and the number of carbon atoms of the alkyl moiety is 1 to 44 (preferably 1 to 30, more preferably 1 to 20, still more preferably 1 to 10, and particularly preferably 1 to 6)). Examples of the aralkyl group include a benzyl group, a phenylethyl group, and a 2-phenylpropan-2-yl group.

A substituent that may substitute $Ar_{11}$, $Ar_{12}$, and $R_{101}$ to $R_{108}$ is preferably a monocyclic group, a fused cyclic group, an alkyl group, a cycloalkyl group, a silyl group, an alkoxy group, a cyano group, or a halogen atom (particularly a fluorine atom), and particularly preferably a monocyclic group or a fused cyclic group. Specific examples of a preferable substituent include those mentioned above in connection with each group in the formulas (1) and (4).

The anthracene derivative shown by the formula (4) is preferably any of the following anthracene derivatives (A), (B), and (C). The anthracene derivative is selected depending on the configuration and the desired properties of the organic EL device to which the anthracene derivative is applied.

(Anthracene derivative (A))

The anthracene derivative (A) is an anthracene derivative shown by the formula (4) wherein $Ar_{11}$ and $Ar_{12}$ are independently a substituted or unsubstituted fused cyclic group having 8 to 50 ring atoms. The anthracene derivative (A) may be an anthracene derivative wherein $Ar_{11}$ and $Ar_{12}$ are identical substituted or unsubstituted fused cyclic groups, or may be an anthracene derivative wherein $Ar_{11}$ and $Ar_{12}$ are different substituted or unsubstituted fused cyclic groups.

The anthracene derivative (A) is preferably an anthracene derivative shown by the formula (4) wherein $Ar_{11}$ and $Ar_{12}$ are different substituted or unsubstituted fused cyclic groups (including a difference in position of a substituent). Specific examples of a preferable fused cyclic group include those mentioned above. A naphthyl group, a phenanthryl group, a benzanthryl group, a 9,9-dimethylfluorenyl group, and a dibenzofuranyl group are particularly preferable as the fused cyclic group.

(Anthracene derivative (B))

The anthracene derivative (B) is an anthracene derivative shown by the formula (4) wherein one of $Ar_{11}$ and $Ar_{12}$ is a substituted or unsubstituted monocyclic group having 5 to 50 ring atoms, and the other of $Ar_{11}$ and $Ar_{12}$ is a substituted or unsubstituted fused cyclic group having 8 to 50 ring atoms.

It is preferable that $Ar_{12}$ be a naphthyl group, a phenanthryl group, a benzanthryl group, a 9,9-dimethylfluorenyl group,

or a dibenzofuranyl group, and $Ar_{11}$ be a phenyl group substituted with a monocyclic group or a fused cyclic group. Specific examples of a preferable monocyclic group and a preferable fused cyclic group include those mentioned above. It is also preferable that $Ar_{12}$ be a fused cyclic group, and $Ar_{11}$ be an unsubstituted phenyl group. In this case, a phenanthryl group, a 9,9-dimethylfluorenyl group, a dibenzofuranyl group, and a benzanthryl group are particularly preferable as the fused cyclic group.

(Anthracene derivative (C))

The anthracene derivative (C) is an anthracene derivative shown by the formula (4) wherein $Ar_{11}$ and $Ar_{12}$ are independently a substituted or unsubstituted monocyclic group having 5 to 50 ring atoms.
It is preferable that $Ar_{11}$ and $Ar_{12}$ be both substituted or unsubstituted phenyl groups.
It is more preferable that $Ar_{11}$ be an unsubstituted phenyl group, and $Ar_{12}$ be a phenyl group substituted with a monocyclic group or a fused cyclic group, or $Ar_{11}$ and $Ar_{12}$ be independently phenyl groups substituted with a monocyclic group or a fused cyclic group.
Specific examples of a preferable monocyclic group and a preferable fused cyclic group as a substituent include those mentioned above. The monocyclic group as a substituent is preferably a phenyl group or a biphenyl group, and the fused cyclic group as a substituent is preferably a naphthyl group, a phenanthryl group, a 9,9-dimethylfluorenyl group, a dibenzofuranyl group, or a benzanthryl group.

(Pyrene derivative)

The pyrene derivative is shown by the following formula (5):

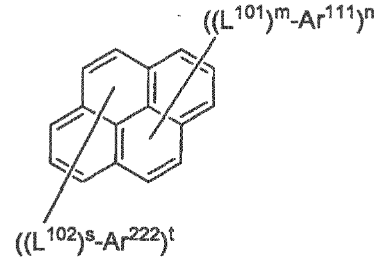

(5)

wherein $Ar^{111}$ and $Ar^{222}$ are independently a substituted or unsubstituted aryl group having 6 to 30 ring carbon atoms, $L^{101}$ and $L^{102}$ are independently a substituted or unsubstituted divalent aryl group having 6 to 30 ring carbon atoms, or a heterocyclic group,
m is an integer from 0 to 1, n is an integer from 1 to 4, s is an integer from 0 to 1, and t is an integer from 0 to 3.
$L^{101}$ or $Ar^{111}$ is bonded to one of positions 1 to 5 of pyrene, and $L^{102}$ or $Ar^{222}$ is bonded to one of positions 6 to 10 of pyrene.
$L^{101}$ and $L^{102}$ in the formula (5) are preferably divalent aryl groups selected from a substituted or unsubstituted phenylene group, a substituted or unsubstituted biphenylene group, a substituted or unsubstituted naphthylene group, a substituted or unsubstituted terphenylene group, a substituted or unsubstituted fluorenylene group, and a combination thereof.
Examples of a substituent include those mentioned above in connection with the formula (1). A substituent that may substitute $L^{101}$ and $L^{102}$ is preferably an alkyl group having 1 to 20 carbon atoms.
m in the formula (5) is preferably an integer from 0 to 1. n in the formula (5) is preferably an integer from 1 to 2. s in the formula (5) is preferably an integer from 0 to 1.
t in the formula (5) is preferably an integer from 0 to 2.
Examples of the aryl group represented by $Ar^{111}$ and $Ar^{222}$ include those mentioned above in connection with the formula (1).
$Ar^{111}$ and $Ar^{222}$ are preferably substituted or unsubstituted aryl groups having 6 to 20 ring carbon atoms, and more preferably substituted or unsubstituted aryl groups having 6 to 16 ring carbon atoms. Specific examples of a preferable aryl group include a phenyl group, a naphthyl group, a phenanthryl group, a fluorenyl group, a biphenyl group, an anthryl group, and a pyrenyl group.
The emitting layer that includes the anthracene derivative shown by the formula (4) or the pyrene derivative shown by the formula (5) is preferably in contact with the barrier layer, the electron-injecting layer, or the electron-transporting layer that includes the imidazopyridine derivative of the invention. When the emitting layer is in contact with the barrier layer, the electron-injecting layer, or the electron-transporting layer that includes the imidazopyridine derivative of the invention, the luminous efficiency can be improved by utilizing the TTF phenomenon.

In the organic EL device of the invention, the emitting layer may include an emitting dopant (phosphorescent dopant and/or fluorescent dopant).
The term "fluorescent dopant" used herein refers to a compound that emits light from singlet excitons. The fluorescent dopant is preferably selected from amine compounds, aromatic compounds, chelate complexes such as a tris(8-quinolinolato)aluminum complex, coumarin derivatives, tetraphenylbutadiene derivatives, bisstyrylarylene derivatives, oxadiazole derivatives, and the like depending on the desired emission color. Among these, styrylamine compounds, styryldiamine compounds, arylamine compounds, aryldiamine compounds, and aromatic compounds are more preferable, and fused polycyclic amine derivatives and aromatic compounds are still more preferable. These fluorescent dopants may be used singly or in combination.

A compound shown by the following formula (12) is preferable as the fused polycyclic amine derivative:

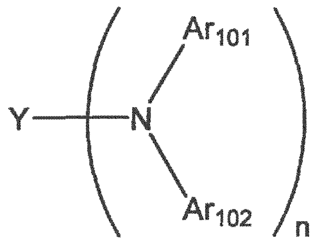

(12)

wherein Y is a substituted or unsubstituted fused aryl group having 10 to 50 ring carbon atoms, and
$Ar_{102}$ and $Ar_{102}$ are independently a substituted or unsubstituted aryl group having 6 to 50 ring carbon atoms, or a substituted or unsubstituted heterocyclic group having 5 to 50 ring atoms.
Specific examples of Y include the above fused aryl groups. Y is preferably a substituted or unsubstituted anthryl group, a substituted or unsubstituted pyrenyl group, or a substituted or unsubstituted chrysenyl group.
n is an integer from 1 to 4, and preferably an integer from 1 to 2.

In the formula (12), examples of the alkyl group, the alkoxy group, the aryl group, the aryloxy group, and the heterocyclic group include those mentioned above.

A fluoranthene compound shown by the following formula (13) is preferable as the aromatic compound:

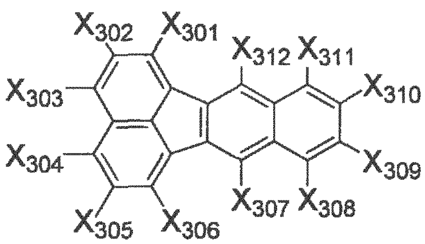

(13)

wherein $X_{301}$ to $X_{306}$ and $X_{308}$ to $X_{311}$ are independently selected from a hydrogen atom, a substituted or unsubstituted aryl group having 6 to 30 ring carbon atoms, a substituted or unsubstituted heterocyclic group having 5 to 30 ring atoms, a substituted or unsubstituted alkyl group having 1 to 10 carbon atoms, a substituted or unsubstituted cycloalkyl group having 3 to 8 ring carbon atoms, a substituted or unsubstituted alkoxy group having 1 to 20 carbon atoms, a substituted or unsubstituted aralkyl group having 7 to 50 carbon atoms, a substituted or unsubstituted aryloxy group having 6 to 50 ring carbon atoms, a substituted or unsubstituted arylthio group having 5 to 50 ring carbon atoms, a substituted or unsubstituted alkoxycarbonyl group having 2 to 50 carbon atoms, an amino group substituted with a substituted or unsubstituted aryl group having 6 to 30 ring carbon atoms, a halogen atom, a cyano group, a nitro group, a hydroxyl group, and a carboxyl group, and
$X_{307}$ and $X_{312}$ are independently selected from a substituted or unsubstituted aryl group having 6 to 30 ring carbon atoms, a substituted or unsubstituted heterocyclic group having 5 to 30 ring atoms, a substituted or unsubstituted alkyl group having 1 to 20 carbon atoms, and a substituted or unsubstituted cycloalkyl group having 3 to 8 ring carbon atoms, provided that $X_{303}$ and $X_{304}$ differ from each other,
adjacent substituents among $X_{301}$ to $X_{312}$ may bond to each other to form a substituted or unsubstituted saturated or unsaturated cyclic structure, and the cyclic structure may be substituted.

$X_{303}$ or $X_{304}$ in the formula (13) is preferably a substituted or unsubstituted aryl group having 6 to 30 ring carbon atoms. A substituent that may substitute each group in the formula (13) is preferably a cyano group or a halogen atom.

Examples of the aryl group, the heterocyclic group, the alkyl group, the cycloalkyl group, the alkoxy group, the aralkyl group, the aryloxy group, the arylthio group, the alkoxycarbonyl group, and the halogen atom include those

mentioned above.

When utilizing the TTF phenomenon, the dopant is preferably a dopant that emits fluorescence having a main peak wavelength of 550 nm or less, and more preferably a blue-emitting dopant.

Note that the term "main peak wavelength" used herein refers to the peak wavelength of the emission spectrum at which the emission intensity becomes a maximum. A main peak wavelength of 550 nm approximately corresponds to green emission. In the region of this wavelength, the luminous efficiency of a fluorescence device is expected to be improved by utilizing the TTF phenomenon. A fluorescence device that emits blue light of a wavelength of 480 nm or less is expected to exhibit further improved luminous efficiency.

The substrate, the anode, the cathode, the hole-injecting layer, the hole-transporting layer, and the like included in the organic EL device of the invention may be appropriately selected and used from among those disclosed in WO2008/023759A1, WO2008/023759A1, WO2009/107596A1, WO2009/081857A1, US2009/0243473A1, US2008/0014464A1, US2009/0021160A1, and the like.

## EXAMPLES

### Synthesis of Intermediates

### Synthesis Example 1

(a) Synthesis of 7,12-diphenylbenzo[k]fluoranthen-3-ylboronic acid

7,12-Diphenylbenzo[k]fluoranthen-3-ylboronic acid was synthesized in accordance with the following scheme.

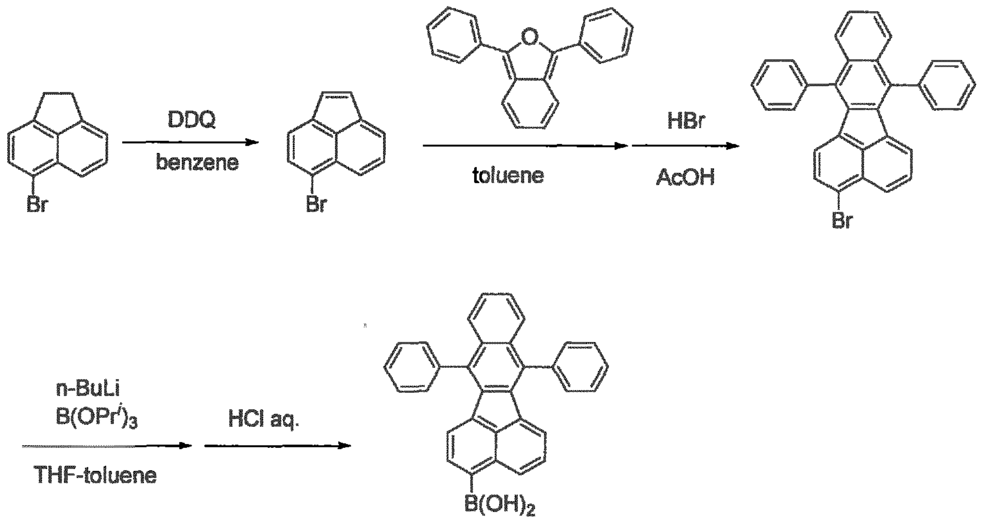


(a-1) Synthesis of 5-bromoacenaphthylene

29.2 g (128.7 mmol) of 2,3-dichloro-5,6-dicyano-1,4-benzoquinone (DDQ) was added to 25.4 g (107.3 mmol) of 5-bromoacenaphthene and 500 ml of dehydrated benzene. The mixture was stirred for 6 hours under heating and reflux. After the addition of 6.0 g (26.4 mmol) of DDQ to the reaction mixture, the mixture was stirred for 4 hours with heating. After allowing the mixture to cool, the precipitate was filtered off, and washed with chloroform. The filtrate was combined and washed with a 10% sodium hydroxide aqueous solution and water. After separation, the organic phase was dried with anhydrous sodium sulfate, and the solvent was evaporated. The residue was dried under reduced pressure to obtain 13.0 g of 5-bromoacenaphthylene as a brown solid (yield: 51.6%).

(a-2) Synthesis of 3-bromo-7,12-diphenylbenzo[k]fluoranthene

A mixture of 14.9 g (55.2 mmol) of 1,3-diphenylisobenzofuran and 12.8 g (55.2 mmol) of 5-bromoacenaphthylene in 50 ml of toluene was stirred for 16 hours under heating and reflux. After evaporating the solvent, 1200 ml of acetic acid was added to the mixture, and the mixture was heated to 80°C. After the addition of 150 ml of a 48% HBr aqueous solution to the mixture, the mixture was stirred at 80°C for 1 hour. After cooling the mixture to room temperature, the precipitate was filtered off, and washed with methanol. The resulting yellow solid was recrystallized from 200 ml of

toluene. The resulting crystal was filtered off to obtain 19.8 g of 3-bromo-7,12-diphenylbenzo[k]fluoranthene as a yellow solid (yield: 74%).

(a-3) Synthesis of 7,12-diphenylbenzo[k]fluoranthen-3-ylboronic acid

30.8 g (64.0 mmol) of 3-bromo-7,12-diphenylbenzo[k]fluoranthene was dissolved in 400 ml of dehydrated tetrahydrofuran and 300 ml of dehydrated toluene. The solution was cooled to -70°C. After the dropwise addition of 44.6 ml (70.4 mmol) of n-butyllithium, the mixture was stirred for 1 hour After the addition of 44.0 ml (192 mmol) of triisopropyl boronic ester, the mixture was heated to room temperature over 2 hours. After the addition of 200 ml of 10% hydrochloric acid, the mixture was stirred for 2 hours. The resulting precipitate was filtered off, washed with toluene, and dried under reduced pressure to obtain 25.14 g of 7,12-diphenylbenzo[k]fluoranthen-3-ylboronic acid as a yellow solid (yield: 88%).

Synthesis Example 2

(b) Synthesis of 4-(7,12-diphenylbenzo[k]fluoranthen-3-yl)phenylboronic acid

4-(7,12-Diphenylbenzo[k]fluoranthen-3-yl)phenylboronic acid was synthesized in accordance with the following scheme.

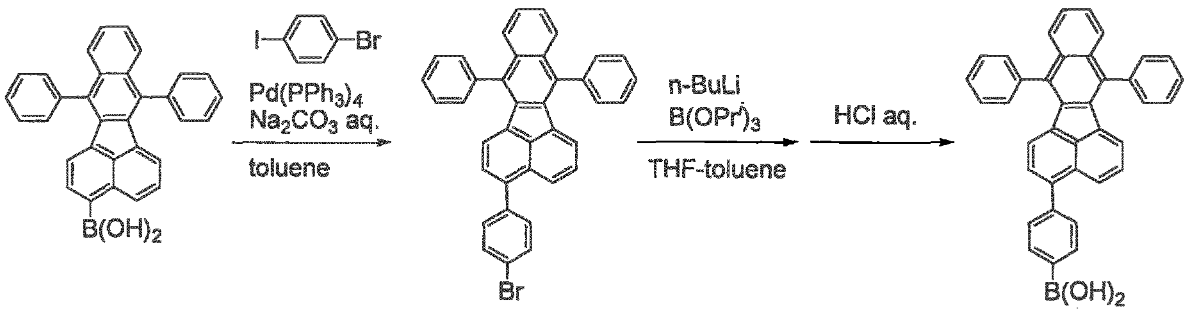


(b-1) Synthesis of 3-(4-bromophenyl)-7,12-diphenylbenzo[k]fluoranthene

A mixture of 1.79 g (4 mmol) of 7,12-diphenylbenzo[k]fluoranthen-3-ylboronic acid, 1.25 g (4.4 mmol) of 4-bromoiodobenzene, 0.14 g (0.12 mmol) of tetrakis(triphenylphosphine)palladium(O), 12 ml of toluene, and 6 ml of a 2 M sodium carbonate aqueous solution was heated and refluxed for 7 hours with stirring in an argon atmosphere. The reaction solution was cooled to room temperature, and extracted with toluene. The organic layer was washed with a saturated sodium chloride solution. After drying the organic layer with anhydrous sodium sulfate, the solvent was evaporated. The residue was purified by silica gel column chromatography to obtain 1.30 g of 3-(4-bromophenyl)-7,12-diphenylbenzo[k]fluoranthene (yield: 58%).

(b-2) Synthesis of 4-(7,12-diphenylbenzo[k]fluoranthen-3-yl)phenylboronic acid

1.30 g (2.32 mmol) of 3-(4-bromophenyl)-7,12-diphenylbenzo[k]fluoranthene was dissolved in 23 ml of dehydrated toluene and 23 ml of dehydrated tetrahydrofuran in an argon atmosphere. The solution was cooled to -70°C. After the dropwise addition of 1.6 ml (2.55 mmol) of n-butyllithium, the mixture was stirred for 1 hour. After the addition of 1.6 ml (6.98 mmol) of triisopropyl boronic ester, the mixture was stirred at -70°C for 1 hour, and heated to room temperature. After the addition of 2 M hydrochloric acid to the reaction solution, the mixture was extracted with toluene. The organic phase was dried over anhydrous sodium sulfate, and the solvent was evaporated. The residue was washed with hexane to obtain 0.75 g of 4-(7,12-diphenylbenzo[k]fluoranthen-3-yl)phenylboronic acid (yield: 62%).

Synthesis Example 3

(c) Synthesis of 2-(4-bromophenyl)-imidazo[1,2-a]pyridine

2-(4-Bromophenyl)-imidazo[1,2-a]pyridine was synthesized in accordance with the following scheme.

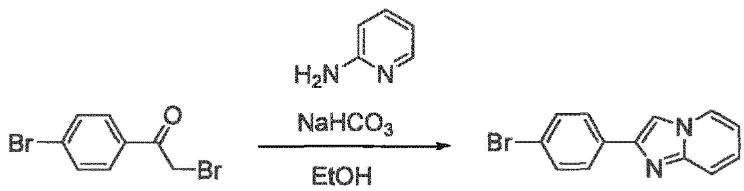


A mixture of 11.1 g (40 mmol) of 4-bromophenacyl bromide, 4.14 g (44 mmol) of 2-aminopyridine, and 5.04 g (60 mmol) of sodium hydrogencarbonate was refluxed in 80 ml of ethanol for 5 hours. After completion of the reaction, the resulting crystal was filtered off, washed with water and acetone, and dried under reduced pressure to obtain 9.54 g of 2-(4-bromophenyl)-imidazo[1,2-a]pyridine (yield: 87%).

Synthesis Example 4

(d) Synthesis of 2-(3-bromophenyl)-imidazo[1,2-a]pyridine

2-(3-Bromophenyl)-imidazo[1,2-a]pyridine was synthesized in the same manner as in Synthesis Example 3, except that 3-bromophenacyl bromide was used instead of 4-bromophenacyl bromide (see the following scheme).

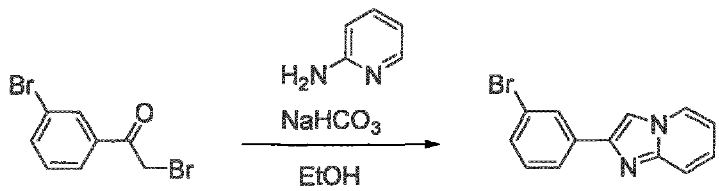


Synthesis Example 5

(e) Synthesis of 6-{4-(imidazo[1,2-a]pyridin-2-yl)phenyl}naphthalen-2-yl trifluoromethanesulfonate

6-{4-(imidazo[1,2-a]pyridin-2-yl)phenyl}naphthalen-2-yl trifluoromethanesulfonate was synthesized in accordance with the following scheme.

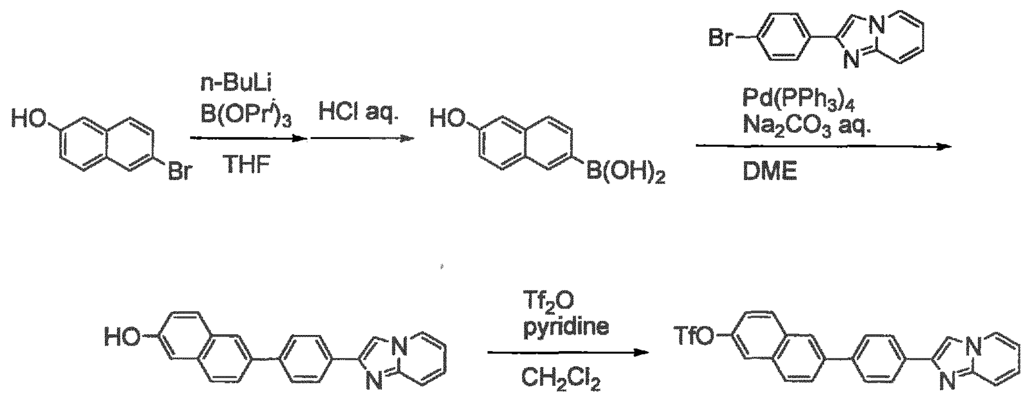


(e-1) Synthesis of 6-hydroxynaphthalen-2-ylboronic acid

5.58 g (25 mmol) of 6-bromo-2-naphthol was dissolved in 125 ml of dehydrated tetrahydrofuran in an argon atmosphere. After cooling the solution to -70°C, 33 ml (55 mmol) of a hexane solution of n-butyllithium was slowly added dropwise to the solution over 30 minutes. The mixture was stirred at -70°C for 1.5 hours. After the addition of 11.5 ml (50 mmol) of triisopropyl borate, the mixture was stirred at -70°C for 30 minutes. The mixture was then stirred for 3 hours while allowing the mixture to slowly return to room temperature. After the addition of 100 ml of 2 M hydrochloric acid to the reaction mixture, the mixture was stirred at room temperature for 2 hours. The reaction solution was then separated, and the aqueous phase was extracted with ethyl acetate. The organic phase was combined, washed with a saturated

sodium chloride solution, and dried with anhydrous sodium sulfate, and the solvent was evaporated. The residue was washed with dichloromethane in a suspended state to obtain 4.02 g of 6-hydroxynaphthalen-2-ylboronic acid (yield: 85%).

(e-2) Synthesis of 6-{4-(imidazo[1,2-a]pyridin-2-yl)phenyl}-2-naphthol

A mixture of 1.57 g (5.73 mmol) of 2-(4-bromophenyl)-imidazo[1,2-a]pyridine, 1.19 g (6.30 mmol) of 6-hydroxynaphthalen-2-ylboronic acid, 0.20 g (0.172 mmol) of tetrakis(triphenylphosphine)palladium(O), 20 ml of 1,2-dimethoxyethane, and 10 ml of a 2 M sodium carbonate aqueous solution was heated and refluxed for 8 hours in an argon atmosphere. The reaction mixture was cooled to room temperature, and neutralized using dilute hydrochloric acid. The resulting solid was filtered off, washed with a methanol-water mixture, and dried. The resulting solid was washed with toluene to obtain 1.45 g of 6-{4-(imidazo[1,2-a]pyridin-2-yl)phenyl}-2-naphthol (yield: 75%).

(e-3) Synthesis of 6-{4-(imidazo[1,2-a]pyridin-2-yl)phenyl}naphthalen-2-yl trifluoromethanesulfonate

A mixture of 1.45 g (4.32 mmol) of 6-{4-(imidazo[1,2-a]pyridin-2-yl)phenyl}-2-naphthol and 40 ml of dichloromethane was cooled to 0°C in an argon atmosphere. After the addition of 1.4 ml (17.3 mmol) of pyridine, 1.4 ml (8.53 mmol) of trifluoromethanesulfonic anhydride was then added, and the mixture was stirred for 10 minutes. The reaction mixture was heated to room temperature, and stirred for 2.5 hours. The reaction mixture was neutralized using 0.5 M hydrochloric acid while cooling the reaction mixture in an ice-water bath, extracted with dichloromethane, washed with a saturated sodium chloride solution, and dried with anhydrous sodium sulfate. The solvent was then evaporated. The residue was purified by silica gel chromatography to obtain 1.87 g of 6-{4-(imidazo[1,2-a]pyridin-2-yl)phenyl}naphthalen-2-yl trifluoromethanesulfonate (yield: 93%).

Synthesis of Imidazopyridine Derivatives

Example 1

A mixture of 1.88 g (4.2 mmol) of 7,12-diphenylbenzo[k]fluoranthen-3-ylboronic acid, 1.09 g (4.0 mmol) of 2-(4-bromophenyl)-imidazo[1,2-a]pyridine, 0.14 g (0.12 mmol) of tetrakis(triphenylphosphine)palladium(0), 12 ml of 1,2-dimethoxyethane, and 6 ml of a 2 M sodium carbonate aqueous solution was refluxed for 7 hours with stirring in an argon atmosphere. The reaction solution was cooled to room temperature. After the addition of water, the mixture was stirred for 1 hour. The resulting solid was filtered off, washed with water and methanol, and dried under reduced pressure. The solid was purified by silica gel chromatography to obtain 2.26 g of a yellow solid. As a result of mass spectrum analysis, it was found that the resulting compound was the following compound 1, which exhibited a molecular weight of 596.23 and m/e of 596. The yield was 95%.

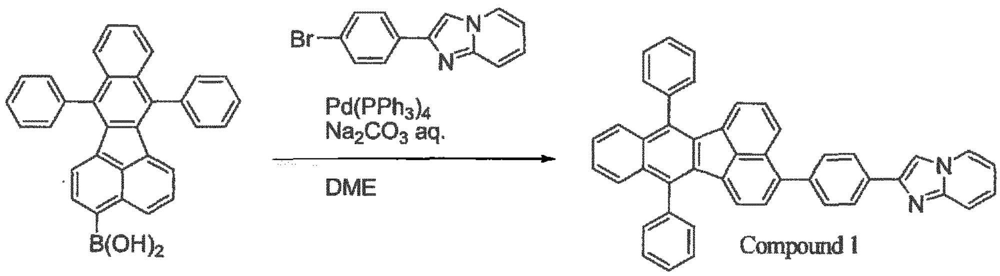


Compound 1

Example 2

A compound was synthesized in the same manner as in Example 1, except that 2-(3-bromophenyl)-imidazo[1,2-a]pyridine was used instead of 2-(4-bromophenyl)-imidazo[1,2,a]pyridine.
As a result of mass spectrum analysis, it was found that the resulting compound was the following compound 2, which exhibited a molecular weight of 596.23 and m/e of 596. The yield was 89%.

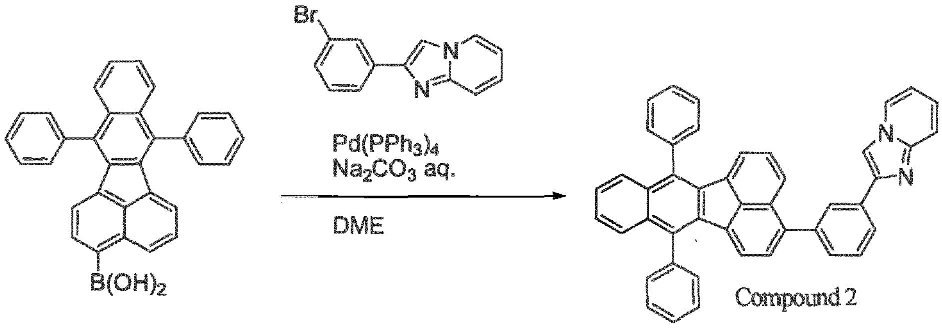

Compound 2

Example 3

A compound was synthesized in the same manner as in Example 1, except that 0.75 g of 4-(7,12-diphenylbenzo[k]fluoranthen-3-yl)phenylboronic acid was used instead of 7,12-diphenylbenzo[k]fluoranthen-3-ylboronic acid. As a result of mass spectrum analysis, it was found that the resulting compound was the following compound 3, which exhibited a molecular weight of 672.26 and m/e of 672. The yield was 60%.

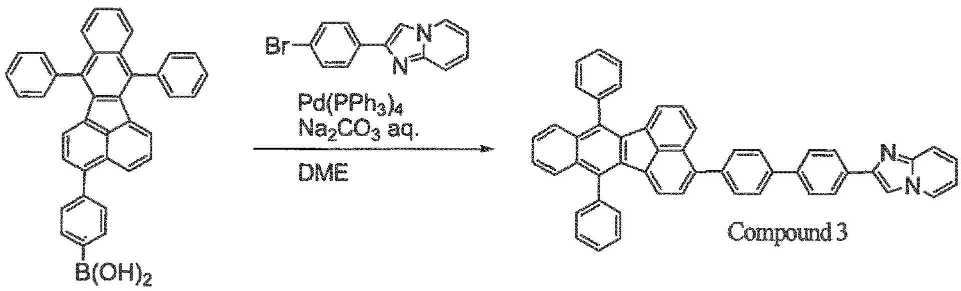

Compound 3

Example 4

A compound was synthesized in the same manner as in Example 1, except that 6-{4-(imidazo[1,2-a]pyridin-2-yl)phenyl}naphthalen-2-yl trifluoromethanesulfonate was used instead of 2-(4-bromophenyl)-imidazo[1,2-a]pyridine. As a result of mass spectrum analysis, it was found that the resulting compound was the following compound 4, which exhibited a molecular weight of 722.27 and m/e of 722. The yield was 75%.

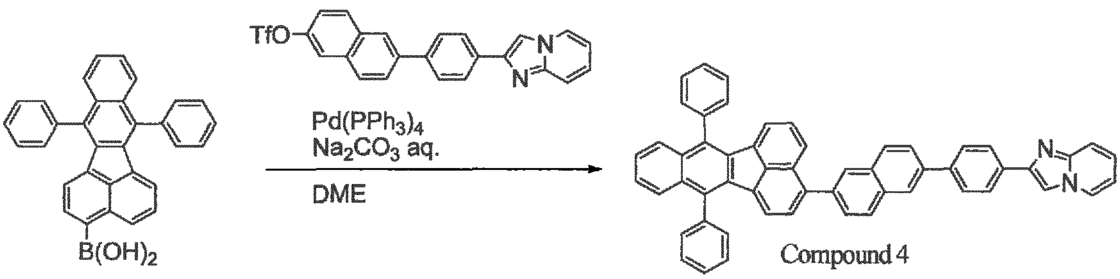

Compound 4

Triplet energy ($E^T$), the ionization potential, and the affinity of the compounds 1 and 2 were evaluated by the following methods. The results are shown in Table 1.

(1) Triplet energy ($E^T$)

The triplet energy was measured using a commercially available instrument "F-4500" (manufactured by Hitachi, Ltd.). The triplet energy ($E^T$) was converted by the following expression.

$$E^T\ (eV)=1239.85/\lambda edge$$

When a tangent to the shorter-wavelength-side rising edge of the phosphorescence spectrum (vertical axis: phosphorescence intensity, horizontal axis: wavelength) is drawn, "λedge" refers to the wavelength at the intersection of the tangent and the horizontal axis. The unit for "λedge" is nm.

(2) Ionization potential

The ionization potential was measured in air using a photoelectron spectrometer ("AC-3" manufactured by Riken Keiki Co., Ltd.). Specifically, light was applied to the compound, and the amount of electrons generated due to carrier separation was measured to determine the ionization potential..

(3) Affinity (Af)

The affinity was calculated from the measured values of the ionization potential and the energy gap. The energy gap was measured from the absorption edge of the absorption spectrum in toluene. Specifically, the absorption spectrum of the compound obtained was measured using a commercially available ultraviolet-visible spectrophotometer, and the energy gap was calculated from the wavelength at the rising edge of the absorption spectrum.
TABLE 1

| | Triplet energy [eV] | Ionization potential [eV] | Affinity [eV] |
|---|---|---|---|
| Compound 1 | 2.1 | 5.9 | 3.1 |
| Compound 2 | 2.1 | 5.9 | 3.1 |

Fabrication of Organic EL Devices

Example 5

The following materials were used to fabricate the organic EL device.

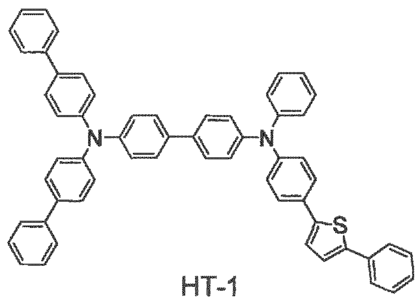

HT-1

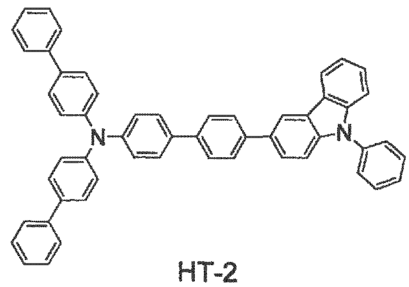

HT-2

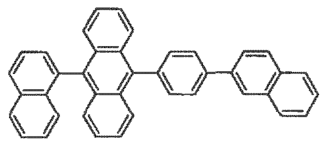

BH-1

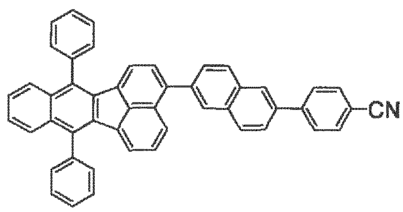

BD-1

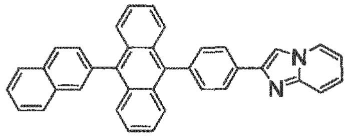

ET-1

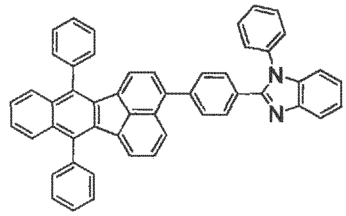

ET-2

A glass substrate of 25 mm by 75 mm by 1.1 mm with an ITO transparent electrode (anode) was subjected to ultrasonic cleaning in isopropyl alcohol for 5 minutes, and cleaning with UV ozone for 30 minutes.
The resultant glass substrate with transparent electrode lines was mounted on the substrate holder of a vacuum deposition apparatus, and the compound HT-1 was deposited on the side of the glass substrate on which the transparent electrode lines were formed so that the transparent electrode was covered, thereby forming a film having a thickness of 50 nm. The resulting HT-1 film functions as a hole-injecting layer. The compound HT-2 was deposited on the HT-1 film to form an HT-2 film having a thickness of 45 nm. The HT-2 film functions as a hole-transporting layer.
The compound BH-1 (host material) and the compound BD-1 (dopant material) were deposited on the HT-2 film in a thickness ratio of 20:1 to obtain an emitting layer having a thickness of 25 nm. The compound 1 was deposited on the emitting layer to form an electron-transporting layer having a thickness of 25 nm on the emitting layer. LiF was then deposited to a thickness of 1 nm. Metal Al was deposited on the LiF film to a thickness of 80 nm. An organic EL device was thus obtained.

The device performance (drive voltage, luminous efficiency, and emission color) at a current density of 10 $mA/cm^2$, and the time required for the luminance to decrease by 5% at a current density of 8 $mA/cm^2$ (95% luminance lifetime) were evaluated using the organic EL device. The results are shown in Table 2.

Examples 6 to 8 and Comparative Examples 1 and 2

Organic EL devices were fabricated in the same manner as in Example 1, except that the compounds 2 to 4, ET-1, and ET-2 were respectively used instead of the compound 1. The resulting organic EL devices were evaluated in the same manner as in Example 1. The results are shown in Table 2.

TABLE 2

| | Electron-transporting layer | Drive voltage [V] | Luminous efficiency [Cd/A] | Emission wavelength [nm] | 95% luminance lifetime [h] |
|---|---|---|---|---|---|
| Example 5 | Compound 1 | 3.5 | 9.0 | Blue | 550 |
| Example 6 | Compound 2 | 3.4 | 8.2 | Blue | 500 |
| Example 7 | Compound 3 | 3.4 | 8.5 | Blue | 490 |
| Example 8 | Compound 4 | 3.4 | 8.1 | Blue | 520 |
| Comparative Example 1 | ET-1 | 3.3 | 7.4 | Blue | 150 |
| Comparative Example 2 | ET-2 | 5.8 | 6.8 | Blue | 450 |

Example 9

A glass substrate of 25 mm by 75 mm by 1.1 mm with an ITO transparent electrode (anode) was subjected to ultrasonic cleaning in isopropyl alcohol for 5 minutes, and cleaning with UV ozone for 30 minutes.
The resultant glass substrate with transparent electrode lines was mounted on the substrate holder of a vacuum deposition apparatus, and the compound HT-1 was deposited on the side of the glass substrate on which the transparent electrode lines were formed so that the transparent electrode was covered, thereby forming a film having a thickness of 50 nm. The resulting HT-1 film functions as a note-injecting layer. The compound HT-2 was deposited on the HT-1 film to form

an HT-2 film having a thickness of 45 nm. The HT-2 film functions as a hole-transporting layer.
The compound BH-1 (host material) and the compound BD-1 (dopant material) were deposited on the HT-2 film in a thickness ratio of 20:1 to obtain an emitting layer having a thickness of 25 nm. The compound 1 and lithium quinolinolate (Liq) were deposited on the emitting layer in a thickness ratio of 1:1 to obtain an electron-transporting layer having a thickness of 25 nm on the emitting layer. Metal Al was deposited on the electron-transporting layer to a thickness of 80 nm, thereby forming a metal cathode. An organic EL device was thus obtained.
The organic EL device was evaluated in the same manner as in Example 5. The results are shown in Table 3.

Example 10 and Comparative Examples 3 and 4

Organic EL devices were fabricated in the same manner as in Example 5, except that the compounds 2, ET-1, and ET-2 were respectively used instead of the compound 1. The resulting organic EL devices were evaluated in the same manner as in Example 5. The results are shown in Table 3.
TABLE 3

| | Electron-transporting layer | Drive voltage [V] | Luminous efficiency [Cd/A] | Emission wavelength [nm] | 95% luminance lifetime [h] |
|---|---|---|---|---|---|
| Example 9 | Compound 1+Liq | 3.5 | 8.4 | Blue | 70 |
| Example 10 | Compound 2+Liq | 3.7 | 8.5 | Blue | 50 |
| Comparative Example 3 | ET-1+Liq | 3.9 | 8.5 | Blue | 13 |
| Comparative Example 4 | ET-2+Liq | 4.6 | 7.7 | Blue | 40 |

As is clear from the results shown in Tables 1 to 3, it was confirmed that a highly efficient low-voltage device that has a long lifetime can be obtained using any of the compounds 1 to 4. It is considered that a decrease in voltage was implemented by introducing the imidazopyridine structure that improves the electron-injecting capability, and a deterioration in the device due to holes that enter the electron-transporting layer was reduced due to the high hole resistance of the benzofluoranthene ring, so that the efficiency and the emission lifetime of the device were improved at the same time.
Since the benzofluoranthene ring has a high affinity, a decrease in drive voltage was also achieved when a codeposition layer of the compound 1 and lithium quinolinolate was used as the electron-transporting layer.
A high luminous efficiency implemented using the compounds 1 to 4 may be due to the effect of the barrier material. The triplet energy of the host material BH-1 calculated in the same manner as the compounds 1 and 2 is 1.83 eV. Since the triplet energy of the compound of the invention is sufficiently larger than 1.83 eV, it is considered that triplet excitons were confined within the emitting layer.

Example 11

A glass substrate of 25 mm by 75 mm by 1.1 mm with an ITO transparent electrode (anode) was subjected to ultrasonic cleaning in isopropyl alcohol for 5 minutes, and cleaning with UV ozone for 30 minutes.
The resultant glass substrate with transparent electrode lines was mounted on the substrate holder of a vacuum deposition apparatus, and the compound HT-1 was deposited on the side of the glass substrate on which the transparent electrode lines were formed so that the transparent electrode was covered, thereby forming a film having a thickness of 50 nm. The resulting HT-1 film functions as a hole-injecting layer. The compound HT-2 was deposited on the HT-1 film to form an HT-2 film having a thickness of 45 nm. The HT-2 film functions as a hole-transporting layer.
The compound BH-1 (host material) and the compound 1 (dopant material) were deposited on the HT-2 film in a thickness ratio of 20:1 to obtain an emitting layer having a thickness of 25 nm. TB-1 was deposited on the emitting layer to form a TB-1 layer having a thickness of 5 nm. The layer functions as a triplet exciton-blocking layer. ET-3 was deposited on the TB-1 layer to form an ET-3 layer having a thickness of 20 nm on the emitting layer. The layer functions as an electron-transporting layer. LiF was then deposited to form a layer having a thickness of 1 nm. Metal Al was deposited on the LiF film to a thickness of 80 nm, thereby forming a metal cathode. An organic EL device was thus obtained.
The structure of the compound TB-1 and the structure of the compound ET-3 are shown below.

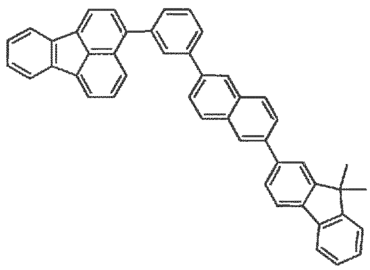

TB-1

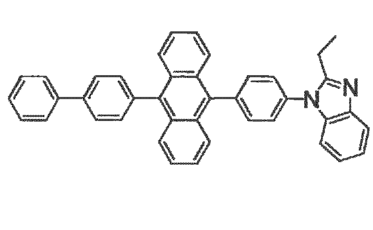

ET-3

The device performance (drive voltage, luminous efficiency, and emission color) at a current density of 10 $mA/cm^2$ was evaluated using the organic EL device. The organic EL device could be driven at 3.5 V, and emitted blue light with a luminous efficiency of 8.7 cd/A.

The compound of the invention may thus be used as a dopant material for a blue fluorescent device, and achieves a high luminous efficiency.

INDUSTRIAL APPLICABILITY

An organic EL device that includes the imidazopyridine derivative according to the invention may be used for a large television display panel, an illumination panel, and the like for which a reduction in power consumption is desired.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The documents described in the specification are incorporated herein by reference in their entirety.

## Claims

1. An imidazopyridine derivative shown by a formula (1),

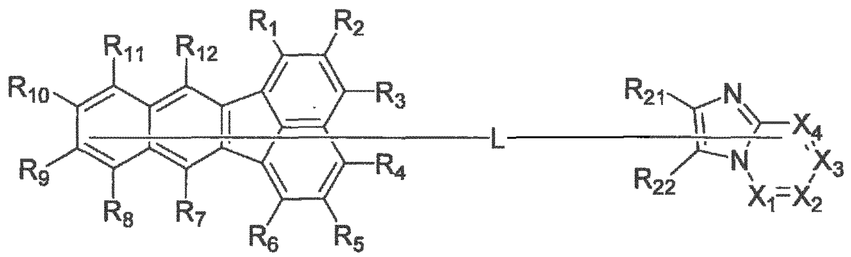

(1)

wherein one of $R_1$ to $R_{12}$ is a single bond that is bonded to L, the remainder of $R_1$ to $R_{12}$ are independently a hydrogen atom, a halogen atom, a cyano group, a substituted or unsubstituted alkyl group having 1 to 10 carbon atoms, a substituted or unsubstituted cycloalkyl group having 3 to 8 ring carbon atoms, a substituted or unsubstituted silyl group, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon atoms, or a substituted or unsubstituted heterocyclic group having 5 to 30 ring atoms,

$X_1$ to $X_4$ are independently a nitrogen atom or a linking group shown by $C(R_{23})$, and/or $X_1$ and $X_2$, $X_2$ and $X_3$, and $X_3$ and $X_4$ respectively bond to each other to form a substituted or unsubstituted aromatic ring having 6 to 18 ring carbon atoms or a substituted or unsubstituted aromatic heteroring having 5 to 18 ring atoms,

provided that a plurality of $R_{23}$ may be either the same or different when a plurality of linking groups shown by $C(R_{23})$ are present,

one of $R_{21}$ to $R_{23}$ is a single bond that is bonded to L, the remainder of $R_{21}$ to $R_{23}$ are independently a hydrogen atom, a halogen atom, a cyano group, a substituted or unsubstituted alkyl group having 1 to 10 carbon atoms, a substituted or unsubstituted cycloalkyl group having 3 to 8 ring carbon atoms, a substituted or unsubstituted silyl group, a substituted or unsubstituted alkoxy group having 1 to 20 carbon atoms, a substituted or unsubstituted aryloxy group having 6 to 20 ring carbon atoms, a substituted or unsubstituted aryl group having 6 to 30 ring carbon

atoms, or a substituted or unsubstituted heterocyclic group having 5 to 30 ring atoms, and
L is a single bond, a substituted or unsubstituted arylene group having 6 to 30 ring carbon atoms, a substituted or unsubstituted divalent heterocyclic group having 5 to 30 ring atoms, or a linking group in which two or three of these divalent groups are bonded via a single bond.

2. The imidazopyridine derivative according to claim 1, the imidazopyridine derivative being shown by a formula (2),

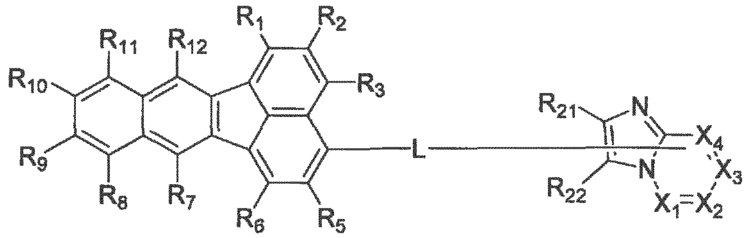

(2)

wherein $X_1$ to $X_4$, $R_1$ to $R_3$, $R_5$ to $R_{12}$, $R_{21}$ to $R_{23}$, and L are the same as defined for the formula (1).

3. The imidazopyridine derivative according to claim 1 or 2, the imidazopyridine derivative being shown by a formula (3),

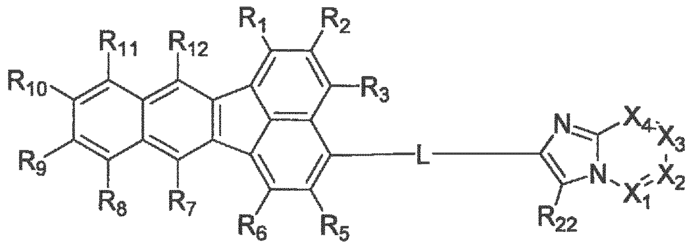

(3)

wherein $X_1$ to $X_4$, $R_1$ to $R_3$, $R_5$ to $R_{12}$, $R_{21}$, $R_{23}$, and L are the same as defined for the formula (1).

4. The imidazopyridine derivative according to any one of claims 1 to 3, wherein $R_7$ and $R_{12}$ are independently an aryl group having 6 to 30 ring carbon atoms.

5. The imidazopyridine derivative according to any one of claims 1 to 4, the imidazopyridine derivative being a material for an organic electroluminescence device.

6. The imidazopyridine derivative according to claim 5, wherein the material is an electron-injecting material or an electron-transporting material.

7. An organic electroluminescence device comprising a cathode, an anode, and one or more organic thin film layers that comprise at least an emitting layer and are between the cathode and the anode, at least one organic thin film layer among the one or more organic thin film layers comprising the imidazopyridine derivative according to any one of claims 1 to 4 either alone or as a component of a mixture.

8. The organic electroluminescence device according to claim 7, wherein the one or more organic thin film layers comprise an electron-injecting layer or an electron-transporting layer, and the electron-injecting layer or the electron-transporting layer comprises the imidazopyridine derivative according to any one of claims 1 to 4 either alone or as a component of a mixture.

9. The organic electroluminescence device according to claim 8, wherein the electron-injecting layer or the electron-transporting layer that comprises the imidazopyridine derivative further comprises a reducing dopant.

10. The organic electroluminescence device according to claim 9, wherein the reducing dopant is one substance or two or more substances selected from the group consisting of alkali metals, alkaline-earth metals, rare earth metals, alkali metal oxides, alkali metal halides, alkaline-earth metal oxides, alkaline-earth metal halides, rare earth metal oxides, rare earth metal halides, organic complexes of alkali metals, organic complexes of alkaline-earth metals,

and organic complexes of rare earth metals.

**11.** The organic electroluminescence device according to claim 10, wherein the reducing dopant is an 8-quinolinol complex of an alkali metal.

FIG.1

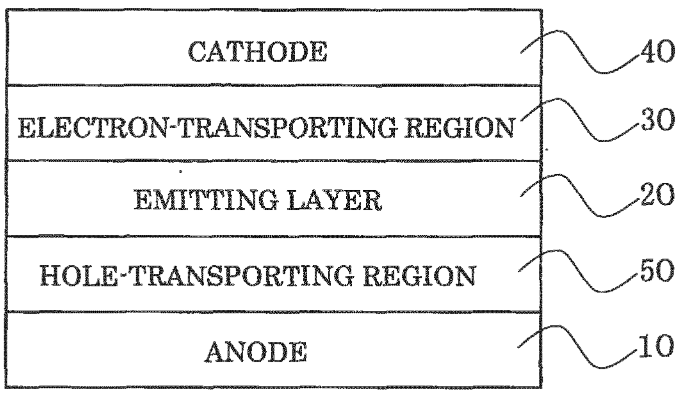
CATHODE
40
ELECTRON-TRANSPORTING REGION
30
EMITTING LAYER
20
HOLE-TRANSPORTING REGION
50
ANODE
10

FIG.2A

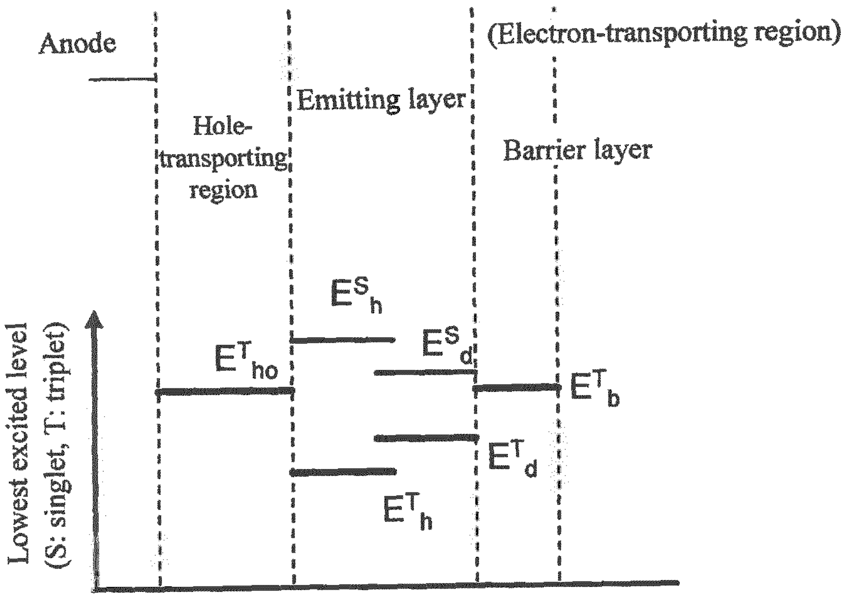
Anode
(Electron-transporting region)
Hole-transporting region
Emitting layer
Barrier layer
Lowest excited level (S: singlet, T: triplet)
$E^S_h$
$E^T_{ho}$
$E^S_d$
$E^T_b$
$E^T_d$
$E^T_h$

FIG.2B

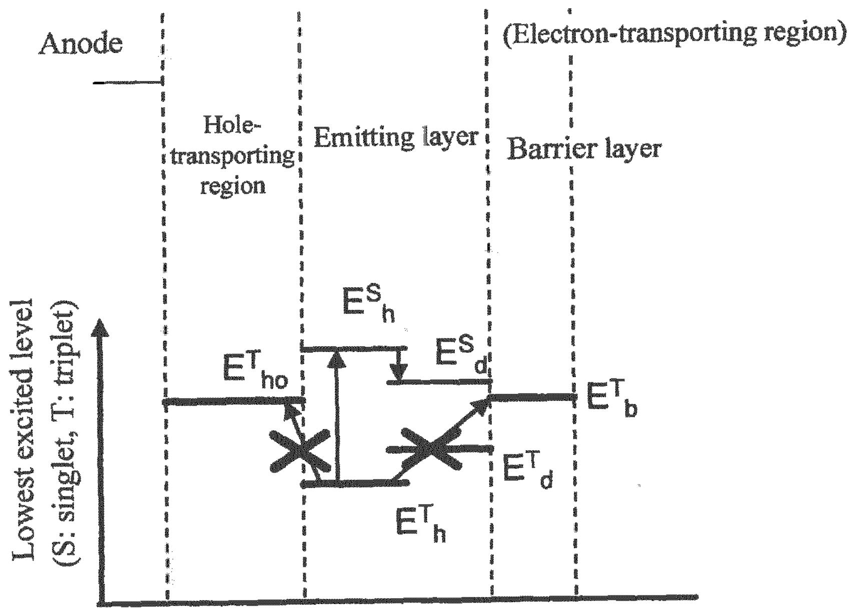
Anode
(Electron-transporting region)
Hole-transporting region
Emitting layer
Barrier layer
Lowest excited level (S: singlet, T: triplet)
$E^S_h$
$E^T_{ho}$
$E^S_d$
$E^T_b$
$E^T_d$
$E^T_h$

## INTERNATIONAL SEARCH REPORT

International application No.
PCT/JP2011/003920

A. CLASSIFICATION OF SUBJECT MATTER
*C07D471/04*(2006.01)i, *H01L51/50*(2006.01)i

According to International Patent Classification (IPC) or to both national classification and IPC

B. FIELDS SEARCHED

Minimum documentation searched (classification system followed by classification symbols)
C07D471/04, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 2004-002297 A (Idemitsu Kosan Co., Ltd.), 08 January 2004 (08.01.2004), claims 1, 4, 5; compounds 11-4, 11-10, 12-5, 12-12, 14-9, 22-9; example 7 & CN 101407492 A & CN 1751024 A & CN 101219987 A & EP 1582516 A1 & JP 2004-217547 A & TW I284488 B & KR 2005091080 A & WO 2004-063159 A1 & US 2006-154105 A1 & US 7867629 B2 | 1-11 |
| Y | JP 2008-069128 A (Idemitsu Kosan Co., Ltd.), 27 March 2008 (27.03.2008), claim 2; paragraph [0023] & US 2008/071122 A1 & WO 2008/032766 A1 & TW 200825153 A | 1-11 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>30 August, 2011 (30.08.11) | Date of mailing of the international search report<br>06 September, 2011 (06.09.11) |
| Name and mailing address of the ISA/<br>Japanese Patent Office<br>Facsimile No. | Authorized officer<br>Telephone No. |

Form PCT/ISA/210 (second sheet) (July 2009)

**INTERNATIONAL SEARCH REPORT**

International application No.
PCT/JP2011/003920

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2009-152529 A (Idemitsu Kosan Co., Ltd.), 09 July 2009 (09.07.2009), claim 2; paragraph [0025] (Family: none) | 1-11 |
| Y | WO 2008/102740 A1 (Idemitsu Kosan Co., Ltd.), 28 August 2008 (28.08.2008), claim 5; paragraph [0038] & EP 2113954 A1 & CN 101617417 A & US 2010/039027 A1 & KR 2010014803 A | 1-11 |
| A | JP 2010-045033 A (Samsung Mobile Display Co., Ltd.), 25 February 2010 (25.02.2010), entire text & US 2010/039029 A1 & EP 2157627 A1 | 1-11 |
| A | WO 2009/148269 A2 (DOOSAN CORP.), 10 December 2009 (10.12.2009), entire text & KR 2009126539 A | 1-11 |
| A | US 2009/149649 A1 (Dong-woo Shin et al.), 11 June 2009 (11.06.2009), formulae 1 to 6, 27, 29, 32, 35 & KR 2009059849 A | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004002297 A
- KR 1020090059849 A
- WO 0914826 A
- JP 2005072012 A
- JP 11345687 A
- WO 2008023759 A1
- WO 2009107596 A1
- WO 2009081857 A1
- US 20090243473 A1
- US 20080014464 A1
- US 20090021160 A1

### Non-patent literature cited in the description

- **S.M. BACHILO et al.** *J. Phys. Chem. A,* 2000, vol. 104, 7711